# EUROPEAN PATENT APPLICATION

(11) **EP 4 714 467 A1**
(43) Date of publication of application: **25.03.2026**
(21) Application number: 24811004.1
(22) Date of filing: 15.05.2024
(51) Int. Cl.: A61K 39/395, A61P 35/00, A61P 35/04, C07K 16/18

(54) **PHARMACEUTICAL COMPOSITION FOR TREATING OR PREVENTING CANCER**

(30) Priority: 19.05.2023 JP 2023083103
(71) Applicant: ISM Co., Ltd., Osaka-shi, Osaka 530-0005 (JP)
(72) Inventor: MIYATO, Mitsuru, Fujisawa-Shi, Kanagawa 251-0052 (JP); OCHIYA, Takahiro, Tokyo 102-0082 (JP)
(74) Representative: D Young & Co LLP
(86) International application number: PCT/JP2024/018033
(87) International publication number: WO 2024/241997

(57) **Abstract**

The present invention relates to a pharmaceutical composition for treating or preventing a cancer, comprising an antibody against coatomer protein complex subunit beta 2 (COPB2) or an antigen binding fragment thereof.

## Description

### Technical Field

The present invention relates to a pharmaceutical composition for treating or preventing a cancer.

### Background Art

Coatomer protein complex subunit beta 2 (COPB2) is one of the non-clathrin-coated vesicular subunits constituting intracellular coatomer which is involved in the membrane transport between the endoplasmic reticulum and the Golgi apparatus, and is a coatomer protein involved in the membrane transport between the endoplasmic reticulum and the Golgi apparatus.

Non-Patent Literature 1 discloses that COPB2, as the coatomer protein, plays an important role in embryonic development or tumor progression, participates in various pathological conditions, and also participates in the control of cancer cell proliferation, survival, invasion, metastasis, or the like, and as such, is under research as an important factor for many cancers.

Non-Patent Literature 2 discloses that COPB2 was highly expressed in glioma; and the high expression of COPB2 correlated with a shortened overall survival time and some poor clinical prognostic variables. Non-Patent Literature 2 further discloses that COPB2 is capable of serving as a potential prognostic biomarker or a target for the immunotherapy for glioma.

Non-Patent Literature 3 discloses that COPB2 is overexpressed in hepatocellular carcinoma (HCC); and the silencing of COPB2 suppressed epithelial-mesenchymal transition and mTOR signal transduction and thereby inhibited the growth, migration, and invasion ability of hepatocellular carcinoma. Non-Patent Literature 3 further discloses that COPB2 is capable of serving as a novel prognostic biomarker or a promising therapeutic target for HCC.

Non-Patent Literature 4 discloses that COPB2 gene is overexpressed in breast cancer tissues; and the downregulation of COPB2 can inhibit the proliferation and cell invasion ability of MDA-MB-231 and BT-549 cell lines.

Non-Patent Literature 5 discloses that COPB2 is highly expressed in lung adenocarcinoma cells; and the silencing of COPB2 suppressed the survival rate and metastasis of cells and drastically increased apoptosis.

Non-Patent Literature 6 discloses that the silencing of COPB2 gene is capable of inhibiting the proliferation of colorectal cancer (CRC) cells and inducing apoptosis via the JNK/c-Jun signal transduction pathway.

Non-Patent Literature 7 discloses that bone marrow mesenchymal stem cell exosome-derived miR-4461 downregulated the expression of COPB2 and suppressed the migration and invasion of large intestinal cancer cells. Non-Patent Literature 9 further discloses that miR-4461 is capable of serving as a potential target for the diagnosis and treatment of large intestinal cancer (colon and rectum).

Non-Patent Literature 8 discloses that the silencing of COPB2 inhibits the proliferation of stomach cancer cells and induces apoptosis via the suppression of the RTK signal transduction pathway.

Non-Patent Literature 9 discloses that COPB2 was identified as mRNA that is downregulated after treatment of stomach cancer cells with chrysin having antitumor characteristics and differentially expressed.

Non-Patent Literature 10 discloses that COPB2 is upregulated in prostate cancer and regulates PC-3 cell proliferation, cell cycles, and apoptosis.

### Citation List

### Non-Patent Literature

Non-Patent Literature 1: Clin Transl Oncol, 2021 (11): 2195-2205
Non-Patent Literature 2: CNS Neuroscience&Therapeutics, 2020, March; 26 (3): 309-318
Non-Patent Literature 3: BioMed Research International; 2021: 6648078
Non-Patent Literature 4: J Cell Mol Med. 2019 Aug; 23 (8): 5235-5245
Non-Patent Literature 5: Transl Cancer Res. 2020 April; 9 (4): 2648-2659
Non-Patent Literature 6: PLoS One, November 19, 2020: e0240106
Non-Patent Literature 7: Biosci Biotechnol Biochem. 2020 Feb; 84 (2): 338-346. 2019 Oct 21
Non-Patent Literature 8: Int J Oncol. 2019 Apr; 54 (4): 1195-1208
Non-Patent Literature 9: Front Oncol. 2021 Jun 3; 11: 651644
Non-Patent Literature 10: Arch Med Res. 2016 Aug; 47 (6): 411-418

### Summary of Invention

### Technical Problem

As mentioned above, the relation of COPB2 to various cancers has been studied and reported. However, these studies have been limited to physiological functional analysis of COPB2 as an intracellular protein by the silencing of COPB2 gene, or the like.

An object of the present invention is to provide a novel pharmaceutical composition for treating or preventing a cancer, targeting COPB2.

### Solution to Problem

The present inventors have conducted diligent studies and consequently completed the present invention by finding that, surprisingly, an antibody against an intracellular protein COPB2 is effective for the treatment of a cancer and the suppression of metastasis.

Specifically, the present invention is as follows.
[1] A pharmaceutical composition for treating or preventing a cancer, comprising an antibody against coatomer protein complex subunit beta 2 (COPB2) or an antigen binding fragment thereof.
[2] The pharmaceutical composition according to [1], wherein the cancer is primary tumor.
[3] The pharmaceutical composition according to [1] or [2] for suppressing cancer recurrence.
[4] The pharmaceutical composition according to any of [1] to [3] for suppressing cancer metastasis.
[5] The pharmaceutical composition according to any of [1] to [4], wherein the cancer is one or more members selected from the group consisting of breast cancer, glioma, gastrointestinal cancer, hepatocellular carcinoma, liver cancer, prostate cancer, lung cancer, and pancreatic cancer.

The present invention also comprises the following aspects.
[A1] A method for treating or preventing a cancer, comprising administering a therapeutically effective amount of an antibody against coatomer protein complex subunit beta 2 (COPB2) or an antigen binding fragment thereof to a patient in need thereof.
[A2] The method according to [A1], wherein the cancer is primary tumor.
[A3] The method according to [A1] or [A2] for suppressing cancer recurrence.
[A4] The method according to any of [A1] to [A3] for suppressing cancer metastasis.
[A5] The method according to any of [A1] to [A4], wherein the cancer is one or more members selected from the group consisting of breast cancer, glioma, gastrointestinal cancer, hepatocellular carcinoma, liver cancer, prostate cancer, lung cancer, and pancreatic cancer.
[B1] An antibody against coatomer protein complex subunit beta 2 (COPB2) or an antigen binding fragment thereof for treating or preventing a cancer.
[B2] The antibody or the antigen binding fragment thereof according to [B1], wherein the cancer is primary tumor.
[B3] The antibody or the antigen binding fragment thereof according to [B1] or [B2] for suppressing cancer recurrence.
[B4] The antibody or the antigen binding fragment thereof according to any of [B1] to [B3] for suppressing cancer metastasis.
[B5] The antibody or the antigen binding fragment thereof according to any of [B1] to [B4], wherein the cancer is one or more members selected from the group consisting of breast cancer, glioma, gastrointestinal cancer, hepatocellular carcinoma, liver cancer, prostate cancer, lung cancer, and pancreatic cancer.
[C1] Use of an antibody against coatomer protein complex subunit beta 2 (COPB2) or an antigen binding fragment thereof for treating or preventing a cancer.
[C2] The use according to [C1], wherein the cancer is primary tumor.
[C3] The use according to [C1] or [C2] for suppressing cancer recurrence.
[C4] The use according to any of [C1] to [C3] for suppressing cancer metastasis.
[C5] The use according to any of [C1] to [C4], wherein the cancer is one or more members selected from the group consisting of breast cancer, glioma, gastrointestinal cancer, hepatocellular carcinoma, liver cancer, prostate cancer, lung cancer, and pancreatic cancer.
[D1] Use of an antibody against coatomer protein complex subunit beta 2 (COPB2) or an antigen binding fragment thereof in the production of a pharmaceutical composition for treating or preventing a cancer.
[D2] The use according to [D1], wherein the cancer is primary tumor.
[D3] The use according to [D1] or [D2] in the production of a pharmaceutical composition for suppressing cancer recurrence.
[D4] The use according to any of [D1] to [D3] in the production of a pharmaceutical composition for suppressing cancer metastasis.
[D5] The use according to any of [D1] to [D4], wherein the cancer is one or more members selected from the group consisting of breast cancer, glioma, gastrointestinal cancer, hepatocellular carcinoma, liver cancer, prostate cancer, lung cancer, and pancreatic cancer.

In any invention of [1] to [5], [A1] to [A5], [B1] to [B5], [C1] to [C5], and [D1] to [D5], the structure of the invention, such as the antibody against coatomer protein complex subunit beta 2 (COPB2) or the antigen binding fragment thereof, can be in any preferred form described as the present embodiment given below. In any invention of [1] to [5], [A1] to [A5], [B1] to [B5], [C1] to [C5], and [D1] to [D5], the structure of the invention may be in a form selected from combinations of arbitrary preferred forms described as the present embodiment given below. Preferred forms include further preferred forms and still further preferred forms.

### Advantageous Effect of Invention

The present invention can provide a pharmaceutical composition for treating or preventing a cancer, comprising an antibody against COPB2 or an antigen binding fragment thereof.

### Brief Description of Drawings

[Figure 1] Figure 1 shows the principle of measurement of an antibody titer after immunization of a mouse with purified human COPB2(650-906) protein.
[Figure 2] Figure 2 shows an antibody titer after two immunizations with purified human COPB2 protein in mice. A high antibody titer of 800000 counts or more was found in all A/Jackson mice, and an antibody titer of 200000 counts or less was found in Balb/c mice.
[Figure 3] Figure 3 shows a study on whether or not COPB2 protein in human cells and mouse cells can be detected by Western blot. Lane 1; 10 µg of a HEK293 cell extract, lane 2; 5 µg of a mouse liver extract, and lane 3; 10 µg of a mouse liver extract.
[Figure 4] Figure 4 shows the amino acid sequences of heavy chain CDR1 to CDR3 and light chain CDR1 to CDR3 (Figure 4A) and the amino acid sequences of a heavy chain variable region and a light chain variable region (Figure 4B) of clone A2-7C6.
[Figure 5] Figure 5 shows the amino acid sequences of heavy chain CDR1 to CDR3 and light chain CDR1 to CDR3 (Figure 5A) and the amino acid sequences of a heavy chain variable region and a light chain variable region (Figure 5B) of clone A2-7D11.
[Figure 6] Figure 6 shows the amino acid sequences of heavy chain CDR1 to CDR3 and light chain CDR1 to CDR3 (Figure 6A) and the amino acid sequences of a heavy chain variable region and a light chain variable region (Figure 6B) of clone A3-8C9.
[Figure 7] Figure 7 shows the amount of luminescence derived from breast cancer cells.
[Figure 8] Figure 8 shows an IVIS measurement image of primary tumor. A lower amount of luminescence was confirmed in a group given an anti-COPB2 antibody than in a control group.
[Figure 9] Figure 9 shows an IVIS measurement image of primary tumor. A lower amount of luminescence was confirmed in a group given an anti-COPB2 antibody than in a control group.
[Figure 10] Figure 10 shows a systemic IVIS measurement image of a breast cancer model mouse after primary tumor excision. A lower amount of luminescence was confirmed in a group given an anti-COPB2 antibody than in a control group.
[Figure 11] Figure 11 shows systemic IVIS measurement results (Figure 11A) and the number of metastatic foci (Figure 11B) of a breast cancer model mouse after primary tumor excision. A lower amount of luminescence as well as a smaller number of metastatic foci was confirmed in a group given an anti-COPB2 antibody than in a control group.

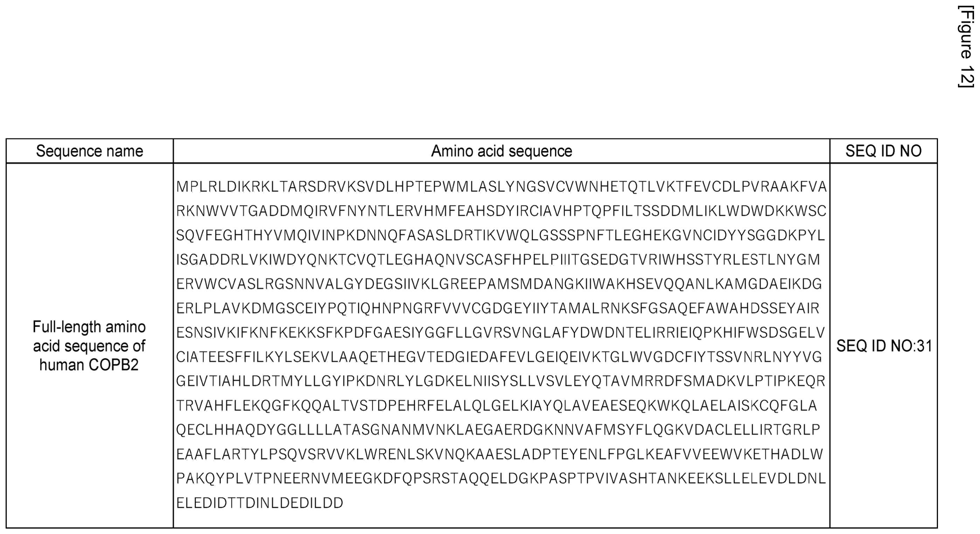

[Figure 12] Figure 12 shows the full-length amino acid sequence of human COPB2.
[Figure 13] Figure 13 shows the amino acid sequences of heavy chain CDR1 to CDR3 and light chain CDR1 to CDR3 (Figure 13A) and the amino acid sequences of a heavy chain variable region and a light chain variable region (Figure 13B) of clone TA201C003a (identification No: #003).
[Figure 14] Figure 14 shows the amino acid sequences of heavy chain CDR1 to CDR3 and light chain CDR1 to CDR3 (Figure 14A) and the amino acid sequences of a heavy chain variable region and a light chain variable region (Figure 14B) of clone TA201B010a (identification No: #010).
[Figure 15] Figure 15 shows an antibody titer after two immunizations with purified human COPB2 protein in TC-mAb mice. Elevation in titer was confirmed in all the immunized individuals by antiserum analysis.
[Figure 16] Figure 16 shows the absorbance of clones top-ranked in secondary screening. Cloning was performed by a limiting dilution method, and a culture supernatant was subjected to ELISA using two immobilized antigens derived from E. *coli* and derived from an animal.
[Figure 17] Figure 17 shows SDS-PAGE results of purified human monoclonal antibodies. Results were compared between a sample treated with a reducing agent DTT (DTT+) and an untreated sample (DTT-).
[Figure 18] Figure 18 shows ELISA results of purified human monoclonal antibodies. The results revealed that clones of identification Nos: #010, #002, and #007 had a high titer. A clone of identification No: #009 was considered unable to be acclimated to a serum-free medium, and only a small amount of a purified antibody was recovered therefrom. The purified antibody was also considered rich in foreign proteins, probably leading to a low titer in ELISA.
[Figure 19] Figure 19 shows the COPB2 reactivity of human monoclonal antibodies by Western blotting. All five antibodies were shown to react with human COPB2. TA201 C003a and TA201 B008a were confirmed to be specific for human COPB2 without reacting with mouse COPB2. TA201B010a was confirmed to react strongly with both human COPB2 and mouse COPB2. Both TA201B007a and TA201C002a were confirmed to react more strongly with mouse COPB2 than with human COPB2.
[Figure 20] Figure 20 shows results of a human isotyping kit. Both purified human monoclonal antibodies of identification Nos: #003 and #010 were confirmed to have IgG1/kappa subclass.
[Figure 21] Figure 21 shows the cytocidal effects (cell viability) and apoptosis inducing effects (caspase 3/7 activity) of anti-COPB2 antibodies against breast cancer cells. Significantly lower viability was confirmed in human breast cancer cells (MDA-MB-231-D3H2LN) cultured in a medium containing a high concentration (20 µM) of TA201B010a clone than in cells cultured in a medium containing only PBS(-). Also, significantly lower caspase 3/7 activity was confirmed in cells cultured in a medium containing a high concentration of TA201B010a clone or TA201 C003a clone.

### Description of Embodiments

Hereinafter, embodiments of the present invention will be described. However, the present invention is not limited by the following descriptions.

### (Anti-COPB2 antibody or antigen binding fragment thereof)

In the present specification, an antibody against coatomer protein complex subunit beta 2 is also referred to as an anti-COPB2 antibody. Description of the anti-COPB2 antibody is also applied to the antigen binding fragment of the anti-COPB2 antibody. Thus, in the present specification, if no mention is made about the antigen binding fragment of the anti-COPB2 antibody, description of the anti-COPB2 antibody should be understood directly as description of the antigen binding fragment of the anti-COPB2 antibody.

COPB2 is a protein that is widely preserved from yeasts to humans. In one aspect, the anti-COPB2 antibody may be an antibody that specifically binds to human COPB2, and the antigen binding fragment of the anti-COPB2 antibody may also be an antigen binding fragment that specifically binds to human COPB2.

The anti-COPB2 antibody can be an antibody that specifically binds to human COPB2 and inhibits the activity of human COPB2. Examples thereof include an antibody that binds to human COPB2 and thereby prevents the human COPB2 from binding to human COPB2 receptor.

The binding site of COPB2 to which the anti-COPB2 antibody binds is not particularly limited, and the anti-COPB2 antibody preferably binds to, for example, one or more peptides having an amino acid sequence in human COPB2.

The present inventors have deduced the reason why the anti-COPB2 antibody is effective for the treatment or prevention of a cancer, as follows, though not intend to particularly limit the reason: when the anti-COPB2 antibody is administered, the anti-COPB2 antibody specifically binds to COPB2 and suppresses the expression of COPB2 or suppresses its binding to human COPB2 receptor. This is considered to suppress cancer growth or to induce the apoptosis of cancer cells. This is also considered to suppress the secretion of exosome from cancer cells.

The term "antibody" as used herein means a molecule having a structure where pairs of two heavy chains (H chains) and two light chains (L chains) stabilized through a disulfide bond are associated with each other.

Two heavy chains are each composed of a heavy chain variable region VH, a heavy chain constant region CH1, CH2, and CH3, and a hinge region positioned between CH1 and CH2, and two light chains are each composed of a light chain variable region VL and a light chain constant region CL. In the antibody, a variable region fragment (Fv) composed of VH and VL is a region that is directly involved in binding to the antigen. An antigen binding region composed of VL, CL, VH, and CH1 is called Fab region, and a region composed of a hinge region, CH2, and CH3 is called Fc region.

In the variable regions, regions that come into direct contact with the antigen are largely variable and are called complementarity-determining regions (CDRs). Relatively less variable moieties other than CDRs are called framework regions (FRs). The light chain and heavy chain variable regions each have three CDRs (heavy chain CDR1 to CDR3 and light chain CDR1 to CDR3).

The anti-COPB2 antibody can be, but not particularly limited to, a monoclonal antibody or a polyclonal antibody.

The term "monoclonal antibody" as used herein means an antibody that is obtained from a population of substantially homogeneous antibodies. The monoclonal antibody is produced by, for example, but not particularly limited to, a hybridoma method, a recombinant DNA method, a phage display method, or a method exploiting a recombinant animal containing the whole or a portion of human immunoglobulin loci, or a combined method thereof.

The term "polyclonal antibody" as used herein means a mixture of different antibodies that recognize a plurality of epitopes on one antigen. The polyclonal antibody is produced, for example, but not particularly limited to, by administering an immunogen comprising the antigen of interest to a mammal (e.g., a rat, a mouse, a rabbit, a bovine, or a monkey) or a bird (e.g., a chicken).

The term "epitope" as used herein means a site (antigenic determinant) that is recognized by the immune system when a certain substance (antigen) induces an antibody thereagainst.

The anti-COPB2 antibody may be an antibody-drug conjugate (ADC) which is a conjugate with another drug.

Examples of another drug for constituting ADC include, but are not particularly limited to, tubulin synthesis inhibitors (anticancer vegetable alkaloids), alkylating agents serving as nucleic acid synthesis inhibitors, topoisomerase inhibitors, antimetabolites, anticancer antibiotics, hormone preparations, platinum-containing drugs, and nonspecific anti-malignant tumor agents.

Examples of the tubulin synthesis inhibitor (anticancer vegetable alkaloid) include, but are not particularly limited to, auristatin, vincristine sulfate, vinblastine sulfate, vindesine sulfate, docetaxel hydrate, paclitaxel, vinorelbine tartrate, and maytansinoid.

Examples of the alkylating agent include, but are not particularly limited to, cyclophosphamide, ifosfamide, melphalan, thiotepa, busulfan, carboquone, dacarbazine, nimustine hydrochloride, and ranimustine.

Examples of the topoisomerase inhibitor include, but are not particularly limited to, camptothecin, topotecan, etoposide, irinotecan hydrochloride, and nogitecan hydrochloride.

Examples of the antimetabolite include, but are not particularly limited to, methotrexate, mercaptopurine, 6-mercaptopurine riboside, fluorouracil (5-FU), tegafur, tegafur uracil, carmofur, doxifluridine, cytarabine ocfosfate, hydroxycarbamide, cytarabine, gemcitabine hydrochloride, fludarabine phosphate, enocitabine, and leucovorin.

Examples of the anticancer antibiotic include, but are not particularly limited to, doxorubicin hydrochloride, idarubicin hydrochloride, epirubicin hydrochloride, pirarubicin hydrochloride, daunorubicin hydrochloride, aclarubicin hydrochloride, mitomycin C, actinomycin D, bleomycin, peplomycin sulfate, neocarzinostatin, and zinostatin stimalamer.

Examples of the hormone preparation include, but are not particularly limited to, estramustine phosphate sodium, flutamide, bicalutamide, goserelin acetate, leuprorelin acetate, tamoxifen citrate, fadrozole hydrochloride hydrate, anastrozole, mepitiostane, epitiostanol, and medroxyprogesterone acetate.

Examples of the platinum-containing drug include, but are not particularly limited to, cisplatin, carboplatin, and nedaplatin.

Examples of the nonspecific anti-malignant tumor agent include, but are not particularly limited to, Krestin, lentinan, sizofiran, and ubenimex.

Other examples of the drug include mitoxantrone hydrochloride, procarbazine hydrochloride, pentostatin, sobuzoxane, tretinoin, L-asparaginase, aceglatone, mitotane, and porfimer sodium.

The term "antigen binding fragment" as used herein means a molecule that comprises a portion of the antibody and is capable of binding to the same antigen as that for the antibody. Examples of the antigen binding fragment include, but are not particularly limited to, Fv, Fab, Fab', Fab'-SH, F(ab')2, diabody, linear antibodies, and single-chain antibody molecules (e.g., scFv).

The term "bispecific antibody" as used herein means a bispecific antibody that can bind to two antigens by means of one antibody, and includes, but is not limited to, an antibody engineered in a bioengineering manner.

The term "nucleotide" or "polynucleotide" as used herein means a nucleic acid and includes, but is not limited to, DNA, RNA, a probe, an oligonucleotide, and a primer.

The term "expression vector" as used herein means an introducing element necessary for expressing the gene of interest, and includes, but is not limited to, a plasmid, a bacteriophage, and the like.

The anti-COPB2 antibody can have a structure known for antibodies and can be any isotype of IgG, IgM, IgA, IgD, and IgE. IgG is an immunoglobulin having γ heavy chains and is produced as a portion of secondary immune response to the antigen. IgM is an immunoglobulin having µ heavy chains and exists as a pentamer in mammals. IgA is an immunoglobulin having α heavy chains. IgD is an immunoglobulin having δ heavy chains. IgE is an immunoglobulin having ε heavy chains.

The anti-COPB2 antibody can be a chimeric antibody, a humanized antibody, a human antibody, or the like. The antigen binding fragment of the anti-COPB2 antibody can be an antigen binding fragment of a chimeric antibody, an antigen binding fragment of a humanized antibody, an antigen binding fragment of a human antibody, or the like. The chimeric antibody is an antibody comprising linked fragments of antibodies derived from different species. The humanized antibody is an antibody comprising nonhuman CDR amino acid sequences grafted in a human antibody. Examples of the humanized antibody include, but are not particularly limited to, antibodies that have heavy chain CDR1 to CDR3 and light chain CDR1 to CDR3 of an antibody prepared by the immunization of a mouse or a rat and have other regions all derived from a human antibody. The term "humanized antibody" may also mean a human chimeric antibody, a mutant human antibody, and a human-type antibody.

In one aspect of the present embodiment, the anti-COPB2 antibody or the antigen binding fragment thereof can comprise any of the following CDR sets (a) to (e):
(a)
   heavy chain CDR1 consisting of the amino acid sequence represented by SEQ ID NO: 1,
   heavy chain CDR2 consisting of the amino acid sequence represented by SEQ ID NO: 2,
   heavy chain CDR3 consisting of the amino acid sequence represented by SEQ ID NO: 3,
   light chain CDR1 consisting of the amino acid sequence represented by SEQ ID NO: 4,
   light chain CDR2 consisting of the amino acid sequence represented by SEQ ID NO: 5, and
   light chain CDR3 consisting of the amino acid sequence represented by SEQ ID NO: 6,
(b)
   heavy chain CDR1 consisting of the amino acid sequence represented by SEQ ID NO: 7,
   heavy chain CDR2 consisting of the amino acid sequence represented by SEQ ID NO: 8,
   heavy chain CDR3 consisting of the amino acid sequence represented by SEQ ID NO: 9,
   light chain CDR1 consisting of the amino acid sequence represented by SEQ ID NO: 10,
   light chain CDR2 consisting of the amino acid sequence represented by SEQ ID NO: 11, and
   light chain CDR3 consisting of the amino acid sequence represented by SEQ ID NO: 12,
(c)
   heavy chain CDR1 consisting of the amino acid sequence represented by SEQ ID NO: 13,
   heavy chain CDR2 consisting of the amino acid sequence represented by SEQ ID NO: 14,
   heavy chain CDR3 consisting of the amino acid sequence represented by SEQ ID NO: 15,
   light chain CDR1 consisting of the amino acid sequence represented by SEQ ID NO: 16,
   light chain CDR2 consisting of the amino acid sequence represented by SEQ ID NO: 17, and
   light chain CDR3 consisting of the amino acid sequence represented by SEQ ID NO: 18,
(d)
   heavy chain CDR1 consisting of the amino acid sequence represented by SEQ ID NO: 32,
   heavy chain CDR2 consisting of the amino acid sequence represented by SEQ ID NO: 33,
   heavy chain CDR3 consisting of the amino acid sequence represented by SEQ ID NO: 34,
   light chain CDR1 consisting of the amino acid sequence represented by SEQ ID NO: 35,
   light chain CDR2 consisting of the amino acid sequence represented by SEQ ID NO: 36, and
   light chain CDR3 consisting of the amino acid sequence represented by SEQ ID NO: 37,
(e)
   heavy chain CDR1 consisting of the amino acid sequence represented by SEQ ID NO: 38,
   heavy chain CDR2 consisting of the amino acid sequence represented by SEQ ID NO: 39,
   heavy chain CDR3 consisting of the amino acid sequence represented by SEQ ID NO: 40,
   light chain CDR1 consisting of the amino acid sequence represented by SEQ ID NO: 41,
   light chain CDR2 consisting of the amino acid sequence represented by SEQ ID NO: 42, and
   light chain CDR3 consisting of the amino acid sequence represented by SEQ ID NO: 43.

The anti-COPB2 antibody or the antigen binding fragment thereof, having any of the CDR sets (a) to (e), is capable of specifically binding to COPB2.

In one aspect of the present embodiment, the anti-COPB2 antibody or the antigen binding fragment thereof can comprise any of the following CDR sets (a1) to (e1):
(a1)
   heavy chain CDR1 consisting of the amino acid sequence represented by SEQ ID NO: 1 or an amino acid sequence differing by having addition, substitution, or deletion of one to several amino acids from the amino acid sequence represented by SEQ ID NO: 1,
   heavy chain CDR2 consisting of the amino acid sequence represented by SEQ ID NO: 2 or an amino acid sequence differing by having addition, substitution, or deletion of one to several amino acids from the amino acid sequence represented by SEQ ID NO: 2,
   heavy chain CDR3 consisting of the amino acid sequence represented by SEQ ID NO: 3 or an amino acid sequence differing by having addition, substitution, or deletion of one to several amino acids from the amino acid sequence represented by SEQ ID NO: 3,
   light chain CDR1 consisting of the amino acid sequence represented by SEQ ID NO: 4 or an amino acid sequence differing by having addition, substitution, or deletion of one to several amino acids from the amino acid sequence represented by SEQ ID NO: 4,
   light chain CDR2 consisting of the amino acid sequence represented by SEQ ID NO: 5 or an amino acid sequence differing by having addition, substitution, or deletion of one to several amino acids from the amino acid sequence represented by SEQ ID NO: 5, and
   light chain CDR3 consisting of the amino acid sequence represented by SEQ ID NO: 6 or an amino acid sequence differing by having addition, substitution, or deletion of one to several amino acids from the amino acid sequence represented by SEQ ID NO: 6,
(b1)
   heavy chain CDR1 consisting of the amino acid sequence represented by SEQ ID NO: 7 or an amino acid sequence differing by having addition, substitution, or deletion of one to several amino acids from the amino acid sequence represented by SEQ ID NO: 7,
   heavy chain CDR2 consisting of the amino acid sequence represented by SEQ ID NO: 8 or an amino acid sequence differing by having addition, substitution, or deletion of one to several amino acids from the amino acid sequence represented by SEQ ID NO: 8,
   heavy chain CDR3 consisting of the amino acid sequence represented by SEQ ID NO: 9 or an amino acid sequence differing by having addition, substitution, or deletion of one to several amino acids from the amino acid sequence represented by SEQ ID NO: 9,
   light chain CDR1 consisting of the amino acid sequence represented by SEQ ID NO: 10 or an amino acid sequence differing by having addition, substitution, or deletion of one to several amino acids from the amino acid sequence represented by SEQ ID NO: 10,
   light chain CDR2 consisting of the amino acid sequence represented by SEQ ID NO: 11 or an amino acid sequence differing by having addition, substitution, or deletion of one to several amino acids from the amino acid sequence represented by SEQ ID NO: 11, and
   light chain CDR3 consisting of the amino acid sequence represented by SEQ ID NO: 12 or an amino acid sequence differing by having addition, substitution, or deletion of one to several amino acids from the amino acid sequence represented by SEQ ID NO: 12,
(c1)
   heavy chain CDR1 consisting of the amino acid sequence represented by SEQ ID NO: 13 or an amino acid sequence differing by having addition, substitution, or deletion of one to several amino acids from the amino acid sequence represented by SEQ ID NO: 13,
   heavy chain CDR2 consisting of the amino acid sequence represented by SEQ ID NO: 14 or an amino acid sequence differing by having addition, substitution, or deletion of one to several amino acids from the amino acid sequence represented by SEQ ID NO: 14,
   heavy chain CDR3 consisting of the amino acid sequence represented by SEQ ID NO: 15 or an amino acid sequence differing by having addition, substitution, or deletion of one to several amino acids from the amino acid sequence represented by SEQ ID NO: 15,
   light chain CDR1 consisting of the amino acid sequence represented by SEQ ID NO: 16 or an amino acid sequence differing by having addition, substitution, or deletion of one to several amino acids from the amino acid sequence represented by SEQ ID NO: 16,
   light chain CDR2 consisting of the amino acid sequence represented by SEQ ID NO: 17 or an amino acid sequence differing by having addition, substitution, or deletion of one to several amino acids from the amino acid sequence represented by SEQ ID NO: 17, and
   light chain CDR3 consisting of the amino acid sequence represented by SEQ ID NO: 18 or an amino acid sequence differing by having addition, substitution, or deletion of one to several amino acids from the amino acid sequence represented by SEQ ID NO: 18,
(d1)
   heavy chain CDR1 consisting of the amino acid sequence represented by SEQ ID NO: 32 or an amino acid sequence differing by having addition, substitution, or deletion of one to several amino acids from the amino acid sequence represented by SEQ ID NO: 32,
   heavy chain CDR2 consisting of the amino acid sequence represented by SEQ ID NO: 33 or an amino acid sequence differing by having addition, substitution, or deletion of one to several amino acids from the amino acid sequence represented by SEQ ID NO: 33,
   heavy chain CDR3 consisting of the amino acid sequence represented by SEQ ID NO: 34 or an amino acid sequence differing by having addition, substitution, or deletion of one to several amino acids from the amino acid sequence represented by SEQ ID NO: 34,
   light chain CDR1 consisting of the amino acid sequence represented by SEQ ID NO: 35 or an amino acid sequence differing by having addition, substitution, or deletion of one to several amino acids from the amino acid sequence represented by SEQ ID NO: 35,
   light chain CDR2 consisting of the amino acid sequence represented by SEQ ID NO: 36 or an amino acid sequence differing by having addition, substitution, or deletion of one to several amino acids from the amino acid sequence represented by SEQ ID NO: 36, and
   light chain CDR3 consisting of the amino acid sequence represented by SEQ ID NO: 37 or an amino acid sequence differing by having addition, substitution, or deletion of one to several amino acids from the amino acid sequence represented by SEQ ID NO: 37,
(e1)
   heavy chain CDR1 consisting of the amino acid sequence represented by SEQ ID NO: 38 or an amino acid sequence differing by having addition, substitution, or deletion of one to several amino acids from the amino acid sequence represented by SEQ ID NO: 38,
   heavy chain CDR2 consisting of the amino acid sequence represented by SEQ ID NO: 39 or an amino acid sequence differing by having addition, substitution, or deletion of one to several amino acids from the amino acid sequence represented by SEQ ID NO: 39,
   heavy chain CDR3 consisting of the amino acid sequence represented by SEQ ID NO: 40 or an amino acid sequence differing by having addition, substitution, or deletion of one to several amino acids from the amino acid sequence represented by SEQ ID NO: 40,
   light chain CDR1 consisting of the amino acid sequence represented by SEQ ID NO: 41 or an amino acid sequence differing by having addition, substitution, or deletion of one to several amino acids from the amino acid sequence represented by SEQ ID NO: 41,
   light chain CDR2 consisting of the amino acid sequence represented by SEQ ID NO: 42 or an amino acid sequence differing by having addition, substitution, or deletion of one to several amino acids from the amino acid sequence represented by SEQ ID NO: 42, and
   light chain CDR3 consisting of the amino acid sequence represented by SEQ ID NO: 43 or an amino acid sequence differing by having addition, substitution, or deletion of one to several amino acids from the amino acid sequence represented by SEQ ID NO: 43.

The anti-COPB2 antibody is preferably an antibody comprising any of the CDR sets (a) to (e) and may be an antibody comprising any of the CDR sets (a1) to (e1). Specifically, the description above should be understood as stating that the antigen binding fragment of the anti-COPB2 antibody is preferably an antigen binding fragment comprising any of the CDR sets (a) to (e) and may be an antigen binding fragment comprising any of the CDR sets (a1) to (e1).

In the CDR sets (a1) to (e1), the heavy chain CDR1 to CDR3 and the light chain CDR1 to CDR3 may be of any of the CDR sets (a) to (e). In the CDR sets (a1) to (e1), the amino acid sequence of any CDR may be an amino acid sequence differing by having addition, substitution, or deletion of one to several amino acids from any of the amino acid sequences represented by SEQ ID NOs: 1 to 6, and the number of CDRs having the amino acid sequence having addition, substitution, or deletion of one to several amino acids can be 1 to 6.

When the anti-COPB2 antibody or the antigen binding fragment thereof has any of the CDR sets (a1) to (e1), any of the CDR sets is a set that has or retains the ability to bind to COPB2.

When the phrase "having addition, substitution, or deletion of one to several amino acids" is used herein, the number of amino acids to be added, substituted, or deleted may be, for example, 1, 2, 3, 4, 5, 6, 7, 8, 9, or 10.

When the phrase "having addition, substitution, or deletion of one to several amino acids" is used concerning the amino acid sequence of CDR, the number of amino acids to be added, substituted, or deleted is not particularly limited as long as the anti-COPB2 antibody has binding activity against COPB2. The number can be set to, for example, 1, 2, 3, or 4. The amino acid sequence of CDR may have "addition, substitution, or deletion of one amino acid", may have "addition, substitution, or deletion of one or two amino acids", may have "addition, or deletion of one to three amino acids", and may have "addition, substitution, or deletion of one to four amino acids". The amino acid sequence of CDR preferably has "addition, substitution, or deletion of one or two amino acids" and preferably has "addition, substitution, or deletion of one amino acid".

The position of addition, substitution, or deletion of an amino acid may be any position in the amino acid sequence of CDR as long as binding activity against COPB2 is retained. The heavy chain and light chain variable regions of an antibody each comprise FR1, CDR1, FR2, CDR2, FR3, CDR3, and FR4 from the N terminus to the C terminus. Particularly, the heavy chain CDR3 is an important moiety that determines the antigen specificity of the antibody. The anti-COPB2 antibody, when having a mutation in CDR, may have addition, substitution, or deletion of one to several amino acids in CDR1 and/or CDR2 except for CDR3 and may have addition, substitution, or deletion of one amino acid therein, as long as the antibody retains antigen specificity.

In one aspect of the present embodiment, the anti-COPB2 antibody or the antigen binding fragment thereof can comprise any of the following CDR sets (a2) to (e2):
(a2)
   heavy chain CDR1 consisting of an amino acid sequence having 80% or higher identity to the amino acid sequence represented by SEQ ID NO: 1,
   heavy chain CDR2 consisting of an amino acid sequence having 80% or higher identity to the amino acid sequence represented by SEQ ID NO: 2,
   heavy chain CDR3 consisting of an amino acid sequence having 80% or higher identity to the amino acid sequence represented by SEQ ID NO: 3,
   light chain CDR1 consisting of an amino acid sequence having 80% or higher identity to the amino acid sequence represented by SEQ ID NO: 4,
   light chain CDR2 consisting of an amino acid sequence having 80% or higher identity to the amino acid sequence represented by SEQ ID NO: 5, and
   light chain CDR3 consisting of an amino acid sequence having 80% or higher identity to the amino acid sequence represented by SEQ ID NO: 6,
(b2)
   heavy chain CDR1 consisting of an amino acid sequence having 80% or higher identity to the amino acid sequence represented by SEQ ID NO: 7,
   heavy chain CDR2 consisting of an amino acid sequence having 80% or higher identity to the amino acid sequence represented by SEQ ID NO: 8,
   heavy chain CDR3 consisting of an amino acid sequence having 80% or higher identity to the amino acid sequence represented by SEQ ID NO: 9,
   light chain CDR1 consisting of an amino acid sequence having 80% or higher identity to the amino acid sequence represented by SEQ ID NO: 10,
   light chain CDR2 consisting of an amino acid sequence having 80% or higher identity to the amino acid sequence represented by SEQ ID NO: 11, and
   light chain CDR3 consisting of an amino acid sequence having 80% or higher identity to the amino acid sequence represented by SEQ ID NO: 12,
(c2)
   heavy chain CDR1 consisting of an amino acid sequence having 80% or higher identity to the amino acid sequence represented by SEQ ID NO: 13,
   heavy chain CDR2 consisting of an amino acid sequence having 80% or higher identity to the amino acid sequence represented by SEQ ID NO: 14,
   heavy chain CDR3 consisting of an amino acid sequence having 80% or higher identity to the amino acid sequence represented by SEQ ID NO: 15,
   light chain CDR1 consisting of an amino acid sequence having 80% or higher identity to the amino acid sequence represented by SEQ ID NO: 16,
   light chain CDR2 consisting of an amino acid sequence having 80% or higher identity to the amino acid sequence represented by SEQ ID NO: 17, and
   light chain CDR3 consisting of an amino acid sequence having 80% or higher identity to the amino acid sequence represented by SEQ ID NO: 18,
(d2)
   heavy chain CDR1 consisting of an amino acid sequence having 80% or higher identity to the amino acid sequence represented by SEQ ID NO: 32,
   heavy chain CDR2 consisting of an amino acid sequence having 80% or higher identity to the amino acid sequence represented by SEQ ID NO: 33,
   heavy chain CDR3 consisting of an amino acid sequence having 80% or higher identity to the amino acid sequence represented by SEQ ID NO: 34,
   light chain CDR1 consisting of an amino acid sequence having 80% or higher identity to the amino acid sequence represented by SEQ ID NO: 35,
   light chain CDR2 consisting of an amino acid sequence having 80% or higher identity to the amino acid sequence represented by SEQ ID NO: 36, and
   light chain CDR3 consisting of an amino acid sequence having 80% or higher identity to the amino acid sequence represented by SEQ ID NO: 37,
(e2)
   heavy chain CDR1 consisting of an amino acid sequence having 80% or higher identity to the amino acid sequence represented by SEQ ID NO: 38,
   heavy chain CDR2 consisting of an amino acid sequence having 80% or higher identity to the amino acid sequence represented by SEQ ID NO: 39,
   heavy chain CDR3 consisting of an amino acid sequence having 80% or higher identity to the amino acid sequence represented by SEQ ID NO: 40,
   light chain CDR1 consisting of an amino acid sequence having 80% or higher identity to the amino acid sequence represented by SEQ ID NO: 41,
   light chain CDR2 consisting of an amino acid sequence having 80% or higher identity to the amino acid sequence represented by SEQ ID NO: 42, and
   light chain CDR3 consisting of an amino acid sequence having 80% or higher identity to the amino acid sequence represented by SEQ ID NO: 43.

The anti-COPB2 antibody is preferably an antibody comprising any of the CDR sets (a) to (e) and may be an antibody comprising any of the CDR sets (a2) to (e2). Specifically, the description above should be understood as stating that the antigen binding fragment of the anti-COPB2 antibody is preferably an antigen binding fragment comprising any of the CDR sets (a) to (e) and may be an antigen binding fragment comprising any of the CDR sets (a2) to (e2).

In the CDR sets (a2) to (e2), the heavy chain CDR1 to CDR3 and the light chain CDR1 to CDR3 may be of any of the CDR sets (a) to (e). In the CDR sets (a2) to (e2), which are not any of the CDR sets (a) to (e), the amino acid sequence of any CDR may be an amino acid sequence having 80% or higher identity to any of the amino acid sequences represented by SEQ ID NOs: 1 to 6, and the number of CDRs having the amino acid sequence having 80% or higher identity can be 1 to 6.

When the anti-COPB2 antibody or the antigen binding fragment thereof has any of the CDR sets (a2) to (e2), any of the CDR sets is a set that has or retains the ability to bind to COPB2.

The phrase "having 80% or higher identity" as used herein means that when an amino acid sequence before mutation is aligned with an amino acid sequence mutated from the amino acid sequence so as to maximize matches, the number of common amino acid residues is 80% or more of the number of amino acids of the amino acid sequence before mutation.

The identity can be, for example, 85% or higher, 90% or higher, 95% or higher, 96% or higher, 97% or higher, 98% or higher, or 99% or higher. The identity may be 100%.

A set of heavy chain CDR1 to CDR3 and light chain CDR1 to CDR3 each consisting of only an amino acid sequence having 100% identity is any of the CDR sets (a) to (e).

In one aspect of the present embodiment, the anti-COPB2 antibody or the antigen binding fragment thereof can be an antibody against coatomer protein complex subunit beta 2 (COPB2) or an antigen binding fragment thereof, comprising any of heavy chain variable regions and/or light chain variable regions (f) to (j) given below.

The heavy chain variable regions and/or the light chain variable regions (f) to (j) given below preferably have any of the CDR sets described above and more preferably have any of the CDR sets (a) to (e).

The case of (f) will be described as an example (the same holds true for (g) to (j)). The heavy chain variable region and/or the light chain variable region (f) is any heavy chain variable region described in (f), is any light chain variable region described in (f), or is any heavy chain variable region described in (f) and any light chain variable region described in (f).

The anti-COPB2 antibody preferably comprises any of the following heavy chain variable regions and light chain variable regions (f) to (j), and, for example, in the case of (f), more preferably comprises any heavy chain variable region (f) and any light chain variable region (f):
(f)
   a heavy chain variable region comprising the amino acid sequence represented by SEQ ID NO: 19;
   a heavy chain variable region comprising an amino acid sequence differing by having addition, substitution, or deletion of one to several amino acids from the amino acid sequence represented by SEQ ID NO: 19; or
   a heavy chain variable region comprising an amino acid sequence having 80% or higher identity to the amino acid sequence represented by SEQ ID NO: 19, and/or
   a light chain variable region comprising the amino acid sequence represented by SEQ ID NO: 20;
   a light chain variable region comprising an amino acid sequence differing by having addition, substitution, or deletion of one to several amino acids from the amino acid sequence represented by SEQ ID NO: 20; or
   a light chain variable region comprising an amino acid sequence having 80% or higher identity to the amino acid sequence represented by SEQ ID NO: 20,
(g)
   a heavy chain variable region comprising the amino acid sequence represented by SEQ ID NO: 21;
   a heavy chain variable region comprising an amino acid sequence differing by having addition, substitution, or deletion of one to several amino acids from the amino acid sequence represented by SEQ ID NO: 21; or
   a heavy chain variable region comprising an amino acid sequence having 80% or higher identity to the amino acid sequence represented by SEQ ID NO: 21, and/or
   a light chain variable region comprising the amino acid sequence represented by SEQ ID NO: 22;
   a light chain variable region comprising an amino acid sequence differing by having addition, substitution, or deletion of one to several amino acids from the amino acid sequence represented by SEQ ID NO: 22; or
   a light chain variable region comprising an amino acid sequence having 80% or higher identity to the amino acid sequence represented by SEQ ID NO: 22,
(h)
   a heavy chain variable region comprising the amino acid sequence represented by SEQ ID NO: 23;
   a heavy chain variable region comprising an amino acid sequence differing by having addition, substitution, or deletion of one to several amino acids from the amino acid sequence represented by SEQ ID NO: 23; or
   a heavy chain variable region comprising an amino acid sequence having 80% or higher identity to the amino acid sequence represented by SEQ ID NO: 23, and/or
   a light chain variable region comprising the amino acid sequence represented by SEQ ID NO: 24;
   a light chain variable region comprising an amino acid sequence differing by having addition, substitution, or deletion of one to several amino acids from the amino acid sequence represented by SEQ ID NO: 24; or
   a light chain variable region comprising an amino acid sequence having 80% or higher identity to the amino acid sequence represented by SEQ ID NO: 24,
(i)
   a heavy chain variable region comprising the amino acid sequence represented by SEQ ID NO: 44;
   a heavy chain variable region comprising an amino acid sequence differing by having addition, substitution, or deletion of one to several amino acids from the amino acid sequence represented by SEQ ID NO: 44; or
   a heavy chain variable region comprising an amino acid sequence having 80% or higher identity to the amino acid sequence represented by SEQ ID NO: 44,
   a light chain variable region comprising the amino acid sequence represented by SEQ ID NO: 45;
   a light chain variable region comprising an amino acid sequence differing by having addition, substitution, or deletion of one to several amino acids from the amino acid sequence represented by SEQ ID NO: 45; or
   a light chain variable region comprising an amino acid sequence having 80% or higher identity to the amino acid sequence represented by SEQ ID NO: 45,
(j)
   a heavy chain variable region comprising the amino acid sequence represented by SEQ ID NO: 46;
   a heavy chain variable region comprising an amino acid sequence differing by having addition, substitution, or deletion of one to several amino acids from the amino acid sequence represented by SEQ ID NO: 46; or
   a heavy chain variable region comprising an amino acid sequence having 80% or higher identity to the amino acid sequence represented by SEQ ID NO: 46,
   a light chain variable region comprising the amino acid sequence represented by SEQ ID NO: 47;
   a light chain variable region comprising an amino acid sequence differing by having addition, substitution, or deletion of one to several amino acids from the amino acid sequence represented by SEQ ID NO: 47; or
   a light chain variable region comprising an amino acid sequence having 80% or higher identity to the amino acid sequence represented by SEQ ID NO: 47.

The heavy chain and light chain variable regions have framework regions (FRs) in addition to CDRs. The amino acid sequence of each FR may be the amino acid sequence itself derived from the FR in each variable region of each immunoglobulin, may be a sequence having a mutation in the amino acid sequence, or may be partially engineered, for example, by introducing a restriction enzyme recognition site into a portion of the amino acid sequence derived from the FR. In the amino acid sequence of the heavy chain and/or light chain variable region, an amino acid mutation may be present in a region other than CDR, i.e., FR, from the viewpoint of less influencing reactivity with the antigen.

The anti-COPB2 antibody may comprise the following CDR set:
heavy chain CDR1 consisting of the amino acid sequence represented by SEQ ID NO: 1,
heavy chain CDR2 consisting of the amino acid sequence represented by SEQ ID NO: 2,
heavy chain CDR3 consisting of the amino acid sequence represented by SEQ ID NO: 3,
light chain CDR1 consisting of the amino acid sequence represented by SEQ ID NO: 4,
light chain CDR2 consisting of the amino acid sequence represented by SEQ ID NO: 5, and
light chain CDR3 consisting of the amino acid sequence represented by SEQ ID NO: 6, and
comprise any following heavy chain variable region and/or any following light chain variable region:
a heavy chain variable region comprising the amino acid sequence represented by SEQ ID NO: 19;
a heavy chain variable region comprising an amino acid sequence differing by having addition, substitution, or deletion of one to several amino acids from the amino acid sequence represented by SEQ ID NO: 19; or
a heavy chain variable region comprising an amino acid sequence having 80% or higher identity to the amino acid sequence represented by SEQ ID NO: 19,
a light chain variable region comprising the amino acid sequence represented by SEQ ID NO: 20;
a light chain variable region comprising an amino acid sequence differing by having addition, substitution, or deletion of one to several amino acids from the amino acid sequence represented by SEQ ID NO: 20; or
a light chain variable region comprising an amino acid sequence having 80% or higher identity to the amino acid sequence represented by SEQ ID NO: 20.

The anti-COPB2 antibody may comprise the following CDR set:
heavy chain CDR1 consisting of the amino acid sequence represented by SEQ ID NO: 7,
heavy chain CDR2 consisting of the amino acid sequence represented by SEQ ID NO: 8,
heavy chain CDR3 consisting of the amino acid sequence represented by SEQ ID NO: 9,
light chain CDR1 consisting of the amino acid sequence represented by SEQ ID NO: 10,
light chain CDR2 consisting of the amino acid sequence represented by SEQ ID NO: 11, and
light chain CDR3 consisting of the amino acid sequence represented by SEQ ID NO: 12, and
comprise any following heavy chain variable region and/or any following light chain variable region:
a heavy chain variable region comprising the amino acid sequence represented by SEQ ID NO: 21;
a heavy chain variable region comprising an amino acid sequence differing by having addition, substitution, or deletion of one to several amino acids from the amino acid sequence represented by SEQ ID NO: 21; or
a heavy chain variable region comprising an amino acid sequence having 80% or higher identity to the amino acid sequence represented by SEQ ID NO: 21,
a light chain variable region comprising the amino acid sequence represented by SEQ ID NO: 22;
a light chain variable region comprising an amino acid sequence differing by having addition, substitution, or deletion of one to several amino acids from the amino acid sequence represented by SEQ ID NO: 22; or
a light chain variable region comprising an amino acid sequence having 80% or higher identity to the amino acid sequence represented by SEQ ID NO: 22.

The anti-COPB2 antibody may comprise the following CDR set:
heavy chain CDR1 consisting of the amino acid sequence represented by SEQ ID NO: 13,
heavy chain CDR2 consisting of the amino acid sequence represented by SEQ ID NO: 14,
heavy chain CDR3 consisting of the amino acid sequence represented by SEQ ID NO: 15,
light chain CDR1 consisting of the amino acid sequence represented by SEQ ID NO: 16,
light chain CDR2 consisting of the amino acid sequence represented by SEQ ID NO: 17, and
light chain CDR3 consisting of the amino acid sequence represented by SEQ ID NO: 18, and
comprise any following heavy chain variable region and/or any following light chain variable region:
a heavy chain variable region comprising the amino acid sequence represented by SEQ ID NO: 23;
a heavy chain variable region comprising an amino acid sequence differing by having addition, substitution, or deletion of one to several amino acids from the amino acid sequence represented by SEQ ID NO: 23; or
a heavy chain variable region comprising an amino acid sequence having 80% or higher identity to the amino acid sequence represented by SEQ ID NO: 23,
a light chain variable region comprising the amino acid sequence represented by SEQ ID NO: 24;
a light chain variable region comprising an amino acid sequence differing by having addition, substitution, or deletion of one to several amino acids from the amino acid sequence represented by SEQ ID NO: 24; or
a light chain variable region comprising an amino acid sequence having 80% or higher identity to the amino acid sequence represented by SEQ ID NO: 24.

The anti-COPB2 antibody may comprise the following CDR set:
heavy chain CDR1 consisting of the amino acid sequence represented by SEQ ID NO: 32,
heavy chain CDR2 consisting of the amino acid sequence represented by SEQ ID NO: 33,
heavy chain CDR3 consisting of the amino acid sequence represented by SEQ ID NO: 34,
light chain CDR1 consisting of the amino acid sequence represented by SEQ ID NO: 35,
light chain CDR2 consisting of the amino acid sequence represented by SEQ ID NO: 36, and
light chain CDR3 consisting of the amino acid sequence represented by SEQ ID NO: 37, and
comprise any following heavy chain variable region and/or any following light chain variable region:
a heavy chain variable region comprising the amino acid sequence represented by SEQ ID NO: 44;
a heavy chain variable region comprising an amino acid sequence differing by having addition, substitution, or deletion of one to several amino acids from the amino acid sequence represented by SEQ ID NO: 44; or
a heavy chain variable region comprising an amino acid sequence having 80% or higher identity to the amino acid sequence represented by SEQ ID NO: 44, and/or
a light chain variable region comprising the amino acid sequence represented by SEQ ID NO: 45;
a light chain variable region comprising an amino acid sequence differing by having addition, substitution, or deletion of one to several amino acids from the amino acid sequence represented by SEQ ID NO: 45; or
a light chain variable region comprising an amino acid sequence having 80% or higher identity to the amino acid sequence represented by SEQ ID NO: 45.

The anti-COPB2 antibody may comprise the following CDR set:
heavy chain CDR1 consisting of the amino acid sequence represented by SEQ ID NO: 38,
heavy chain CDR2 consisting of the amino acid sequence represented by SEQ ID NO: 39,
heavy chain CDR3 consisting of the amino acid sequence represented by SEQ ID NO: 40,
light chain CDR1 consisting of the amino acid sequence represented by SEQ ID NO: 41,
light chain CDR2 consisting of the amino acid sequence represented by SEQ ID NO: 42, and
light chain CDR3 consisting of the amino acid sequence represented by SEQ ID NO: 43, and
comprise any following heavy chain variable region and/or any following light chain variable region:
a heavy chain variable region comprising the amino acid sequence represented by SEQ ID NO: 46;
a heavy chain variable region comprising an amino acid sequence differing by having addition, substitution, or deletion of one to several amino acids from the amino acid sequence represented by SEQ ID NO: 46; or
a heavy chain variable region comprising an amino acid sequence having 80% or higher identity to the amino acid sequence represented by SEQ ID NO: 46, and/or
a light chain variable region comprising the amino acid sequence represented by SEQ ID NO: 47;
a light chain variable region comprising an amino acid sequence differing by having addition, substitution, or deletion of one to several amino acids from the amino acid sequence represented by SEQ ID NO: 47; or
a light chain variable region comprising an amino acid sequence having 80% or higher identity to the amino acid sequence represented by SEQ ID NO: 47.

In the anti-COPB2 antibody, regions other than CDRs when the CDRs are defined or regions other than the variable regions when a heavy chain and/or light chain variable region are defined may be selected from regions of usual antibodies as long as the anti-COPB2 antibody has the ability to bind to COPB2.

The heavy chain and light chain variable regions in the anti-COPB2 antibody may have amino acid sequences differing from the amino acid sequences represented by SEQ ID NOs: 19 to 24 and 44 to 47 by lacking amino acids constituting a signal sequence.

Specifically, the anti-COPB2 antibody may comprise
a heavy chain variable region comprising an amino acid sequence differing from the amino acid sequence represented by any of SEQ ID NOs: 19, 21, 23, 44, and 46 by lacking amino acids constituting a signal sequence, and/or
a light chain variable region comprising an amino acid sequence differing from the amino acid sequence represented by any of SEQ ID NOs: 20, 22, 24, 45, and 47 by lacking amino acids constituting a signal sequence.

The heavy chain or light chain variable region comprising an amino acid sequence having addition, substitution, or deletion of one to several amino acids can also be a heavy chain or light chain variable region comprising an amino acid sequence differing by having addition, substitution, or deletion of one to several amino acids from an amino acid sequence differing from the amino acid sequence represented by any of SEQ ID NOs: 19 to 24 and 44 to 47 by lacking amino acids constituting a signal sequence.

The heavy chain or light chain variable region having an amino acid sequence having 80% or higher identity can also be a heavy chain or light chain variable region comprising an amino acid sequence having 80% or higher identity to an amino acid sequence differing from the amino acid sequence represented by any of SEQ ID NOs: 19 to 24 and 44 to 47 by lacking amino acids constituting a signal sequence.

The signal sequence corresponds to approximately 20 amino acids from an N-terminal amino acid in the heavy chain and light chain variable regions.

The amino acid sequence corresponding to the signal sequence is as follows:
positions 1 to 28 of SEQ ID NO: 19,
positions 1 to 36 of SEQ ID NO: 20,
positions 1 to 28 of SEQ ID NO: 21,
positions 1 to 22 of SEQ ID NO: 22,
positions 1 to 19 of SEQ ID NO: 23,
positions 1 to 19 of SEQ ID NO: 24,
positions 1 to 19 of SEQ ID NO: 44,
positions 1 to 20 of SEQ ID NO: 45,
positions 1 to 19 of SEQ ID NO: 46, and
positions 1 to 22 of SEQ ID NO: 47.

Thus, the anti-COPB2 antibody or the antigen binding fragment thereof, comprising any of the heavy chain variable regions and/or the light chain variable regions (f) to (j) may be an anti-COPB2 antibody or an antigen binding fragment thereof, comprising any of the following heavy chain variable regions and/or light chain variable regions (f1) to (j1) as an antibody or an antigen binding fragment thereof comprising no signal sequence:
(f1)
   a heavy chain variable region comprising the amino acid sequence represented by positions 29 to 145 of SEQ ID NO: 19 (SEQ ID NO: 25);
   a heavy chain variable region comprising an amino acid sequence differing by having addition, substitution, or deletion of one to several amino acids from the amino acid sequence represented by positions 29 to 145 of SEQ ID NO: 19; or
   a heavy chain variable region comprising an amino acid sequence having 80% or higher identity to the amino acid sequence represented by positions 29 to 145 of SEQ ID NO: 19, and/or
   a light chain variable region comprising the amino acid sequence represented by positions 37 to 143 of SEQ ID NO: 20 (SEQ ID NO: 26);
   a light chain variable region comprising an amino acid sequence differing by having addition, substitution, or deletion of one to several amino acids from the amino acid sequence represented by positions 37 to 143 of SEQ ID NO: 20; or
   a light chain variable region comprising an amino acid sequence having 80% or higher identity to the amino acid sequence represented by positions 37 to 143 of SEQ ID NO: 20,
(g1)
   a heavy chain variable region comprising the amino acid sequence represented by positions 29 to 145 of SEQ ID NO: 21 (SEQ ID NO: 27);
   a heavy chain variable region comprising an amino acid sequence differing by having addition, substitution, or deletion of one to several amino acids from the amino acid sequence represented by positions 29 to 145 of SEQ ID NO: 21; or
   a heavy chain variable region comprising an amino acid sequence having 80% or higher identity to the amino acid sequence represented by positions 29 to 145 of SEQ ID NO: 21, and/or
   a light chain variable region comprising the amino acid sequence represented by positions 23 to 129 of SEQ ID NO: 22 (SEQ ID NO: 28);
   a light chain variable region comprising an amino acid sequence differing by having addition, substitution, or deletion of one to several amino acids from the amino acid sequence represented by positions 23 to 129 of SEQ ID NO: 22; or
   a light chain variable region comprising an amino acid sequence having 80% or higher identity to the amino acid sequence represented by positions 23 to 129 of SEQ ID NO: 22,
(h1)
   a heavy chain variable region comprising the amino acid sequence represented by positions 20 to 134 of SEQ ID NO: 23 (SEQ ID NO: 29);
   a heavy chain variable region comprising an amino acid sequence differing by having addition, substitution, or deletion of one to several amino acids from the amino acid sequence represented by positions 20 to 134 of SEQ ID NO: 23; or
   a heavy chain variable region comprising an amino acid sequence having 80% or higher identity to the amino acid sequence represented by positions 20 to 134 of SEQ ID NO: 23, and/or
   a light chain variable region comprising the amino acid sequence represented by positions 20 to 131 of SEQ ID NO: 24 (SEQ ID NO: 30);
   a light chain variable region comprising an amino acid sequence differing by having addition, substitution, or deletion of one to several amino acids from the amino acid sequence represented by positions 20 to 131 of SEQ ID NO: 24; or
   a light chain variable region comprising an amino acid sequence having 80% or higher identity to the amino acid sequence represented by positions 20 to 131 of SEQ ID NO: 24,
(i1)
   a heavy chain variable region comprising the amino acid sequence represented by positions 20 to 146 of SEQ ID NO: 44 (SEQ ID NO: 48);
   a heavy chain variable region comprising an amino acid sequence differing by having addition, substitution, or deletion of one to several amino acids from the amino acid sequence represented by positions 20 to 146 of SEQ ID NO: 44; or
   a heavy chain variable region comprising an amino acid sequence having 80% or higher identity to the amino acid sequence represented by positions 20 to 146 of SEQ ID NO: 44, and/or
   a light chain variable region comprising the amino acid sequence represented by positions 21 to 132 of SEQ ID NO: 45 (SEQ ID NO: 49);
   a light chain variable region comprising an amino acid sequence differing by having addition, substitution, or deletion of one to several amino acids from the amino acid sequence represented by positions 21 to 132 of SEQ ID NO: 45; or
   a light chain variable region comprising an amino acid sequence having 80% or higher identity to the amino acid sequence represented by positions 21 to 132 of SEQ ID NO: 45,
(j1)
   a heavy chain variable region comprising the amino acid sequence represented by positions 20 to 144 of SEQ ID NO: 46 (SEQ ID NO: 50);
   a heavy chain variable region comprising an amino acid sequence differing by having addition, substitution, or deletion of one to several amino acids from the amino acid sequence represented by positions 20 to 144 of SEQ ID NO: 46; or
   a heavy chain variable region comprising an amino acid sequence having 80% or higher identity to the amino acid sequence represented by positions 20 to 144 of SEQ ID NO: 46, and/or
   a light chain variable region comprising the amino acid sequence represented by positions 23 to 129 of SEQ ID NO: 47 (SEQ ID NO: 51);
   a light chain variable region comprising an amino acid sequence differing by having addition, substitution, or deletion of one to several amino acids from the amino acid sequence represented by positions 23 to 129 of SEQ ID NO: 47; or
   a light chain variable region comprising an amino acid sequence having 80% or higher identity to the amino acid sequence represented by positions 23 to 129 of SEQ ID NO: 47.

The anti-COPB2 antibody or the antigen binding fragment thereof, comprising any of the heavy chain variable regions and/or the light chain variable regions (f1) to (j1) is preferably an antibody or an antigen binding fragment thereof comprising any of the CDR sets (a) to (e), (a1) to (e1), or (a2) to (e2).

Specifically, the anti-COPB2 antibody or the antigen binding fragment thereof, comprising any of heavy chain variable regions and/or light chain variable regions (f1) to (j1), when being an anti-COPB2 antibody or an antigen binding fragment thereof, comprising the heavy chain variable region and/or the light chain variable region (f1), preferably comprises any of the CDR set (a), (a1), or (a2) and more preferably comprises the CDR set (a);
when being an anti-COPB2 antibody or an antigen binding fragment thereof, comprising the heavy chain variable region and/or the light chain variable region (g1), preferably comprises any of the CDR set (b), (b1), or (b2) and more preferably comprises the CDR set (b);
when being an anti-COPB2 antibody or an antigen binding fragment thereof, comprising the heavy chain variable region and/or the light chain variable region (h1), preferably comprises any of the CDR set (c), (c1), or (c2) and more preferably comprises the CDR set (c);
when being an anti-COPB2 antibody or an antigen binding fragment thereof, comprising the heavy chain variable region and/or the light chain variable region (i1), preferably comprises any of the CDR set (d), (d1) or (d2) and more preferably comprises the CDR set (d); and
when being an anti-COPB2 antibody or an antigen binding fragment thereof, comprising the heavy chain variable region and/or the light chain variable region (j1), preferably comprises any of the CDR set (e), (e1) or (e2) and more preferably comprises the CDR set (e).

Such an antibody or an antigen binding fragment thereof can be an anti-COPB2 antibody or an antigen binding fragment thereof, comprising any of the heavy chain variable regions and/or the light chain variable regions (f1) to (j1) and comprising any of the CDR sets (a) to (e).

The description of the heavy chain variable regions and/or the light chain variable regions (f) to (j) should be understood as description of the heavy chain variable regions and/or the light chain variable regions (f1) to (j1). In the following description of a nucleic acid, an expression vector, a transformant, a production method, a kit, and the like in the present specification, the description of the heavy chain variable regions and/or the light chain variable regions (f) to (j) should also be understood as description of the heavy chain variable regions and/or the light chain variable regions (f1) to (j1).

In one aspect of the present embodiment, the anti-COPB2 antibody or the antigen binding fragment thereof is not particularly limited as long as the anti-COPB2 antibody or the antigen binding fragment thereof is derived from an antibody against COPB2. The anti-COPB2 antibody or the antigen binding fragment thereof may be a commercially available anti-COPB2 antibody or an antigen binding fragment thereof. The anti-COPB2 antibody is not particularly limited as long as the anti-COPB2 antibody is generally available. The anti-COPB2 antibody may be obtained as, for example, an anti-COPB2 monoclonal antibody manufactured by Novus Biologicals, LLC (product name: COPB2 Antibody (M3A5), product code: NB600-102, etc.), an anti-COPB2 polyclonal antibody manufactured by Bethyl Laboratories, Inc. (product name: COPB2 Polyclonal Antibody, catalog No: Cat# A304-522A, Cat# A304-523A, Cat# A304-522A-M, Cat# A304-523A-M, etc.), an anti-COPB2 polyclonal antibody manufactured by Invitrogen Corp. (product name: COPB2 Polyclonal Antibody, catalog No: Catalog # PA5-77106, # PA5-96557, # PA5-49113, # PA5-57852, # PA5-66144, etc.), an anti-COPB2 antibody manufactured by Assay Genie (product name: Anti-COPB2 Antibody, catalog No: PACO44494, CAB7036, etc.), an anti-COPB2 polyclonal antibody manufactured by Beijing Solarbio Science & Technology Co., Ltd. (product name: Anti-COPB2 Polyclonal Antibody, catalog No: K110122P-SAlexa Fluor 633, etc.), an anti-COPB2 polyclonal antibody manufactured by Abcam plc (product name: Anti-COPB2 Antibody, product code: ab229639, ab2915, ab192924, etc.), or an anti-COPB2 antibody manufactured by Atlas Antibodies AB (product name: Anti-COPB2 antibody produced in rabbit, product No: HPA036867, HPA058180, etc.).

The anti-COPB2 antibody or the antigen binding fragment thereof may comprise an amino acid sequence having 80% or higher identity to the amino acid sequence of each commercially available anti-COPB2 antibody.

In one aspect of the present embodiment, the antigen of the anti-COPB2 antibody or the antigen binding fragment thereof is not particularly limited as long as the antigen is derived from the amino acid sequence of COPB2. The amino acid sequence of the antigen is not particularly limited and is, for example, as follows:
the full-length amino acid sequence of human COPB2 (SEQ ID NO: :31);
an amino acid sequence corresponding to positions 286 to 380 in the amino acid sequence of human COPB2;
an amino acid sequence corresponding to positions 494 to 511 in the amino acid sequence of human COPB2;
an amino acid sequence corresponding to positions 650 to 906 in the amino acid sequence of human COPB2;
an amino acid sequence corresponding to positions 657 to 906 in the amino acid sequence of human COPB2;
an amino acid sequence corresponding to positions 707 to 786 in the amino acid sequence of human COPB2;
an amino acid sequence corresponding to positions 800 to 850 in the amino acid sequence of human COPB2;
an amino acid sequence corresponding to positions 814 to 843 in the amino acid sequence of human COPB2;
an amino acid sequence corresponding to positions 850 to 906 in the amino acid sequence of human COPB2; and
an amino acid sequence corresponding to positions 856 to 906 in the amino acid sequence of human COPB2.

Among them, the amino acid sequence of the antigen is, but not particularly limited to, preferably an amino acid sequence consisting of 25 to 257 consecutive amino acid residues selected from an amino acid sequence corresponding to positions 650 to 906 in the amino acid sequence of human COPB2, more preferably any of the following amino acid sequences:
an amino acid sequence corresponding to positions 650 to 906 in the amino acid sequence of human COPB2;
an amino acid sequence corresponding to positions 850 to 906 in the amino acid sequence of human COPB2; and
an amino acid sequence corresponding to positions 856 to 906 in the amino acid sequence of human COPB2.

The anti-COPB2 antibody or the antigen binding fragment thereof can be an antibody against an antigen having the amino acid sequence represented by SEQ ID NO: :31, or an antigen binding fragment thereof.

The anti-COPB2 antibody or the antigen binding fragment thereof can be an antibody against an antigen having an amino acid sequence from positions 286 to 380, an amino acid sequence from positions 494 to 511, an amino acid sequence from positions 650 to 906, an amino acid sequence from positions 657 to 906, an amino acid sequence from positions 707 to 786, an amino acid sequence from positions 800 to 850, an amino acid sequence from positions 814 to 843, an amino acid sequence from positions 850 to 906, or an amino acid sequence from positions 856 to 906 in the amino acid sequence represented by SEQ ID NO: :31, or an antigen binding antibody thereof. The anti-COPB2 antibody or the antigen binding fragment thereof can be an antibody against an antigen having an amino acid sequence from positions 650 to 906, an amino acid sequence from positions 657 to 906, an amino acid sequence from positions 707 to 786, an amino acid sequence from positions 800 to 850, an amino acid sequence from positions 814 to 843, an amino acid sequence from positions 850 to 906, or an amino acid sequence from positions 856 to 906 in the amino acid sequence represented by SEQ ID NO: :31, or an antigen binding fragment thereof, and can be an antibody against an antigen having an amino acid sequence from positions 650 to 906, an amino acid sequence from positions 850 to 906, or an amino acid sequence from positions 856 to 906 in the amino acid sequence represented by SEQ ID NO: :31, or an antigen binding fragment thereof.

### (Method for producing anti-COPB2 antibody or antigen binding fragment thereof)

The method for producing the anti-COPB2 antibody or the antigen binding fragment thereof is not particularly limited. In the case of, for example, a monoclonal antibody, the method can involve isolating antibody-producing cells from a nonhuman mammal immunized with COPB2 or its fragment, fusing the cells with myeloma cells or the like to prepare hybridomas, and purifying antibodies produced from the hybridomas. A polyclonal antibody may be obtained from the serum of an animal immunized with COPB2 or its fragment.

In the case of preparing the anti-COPB2 antibody or the antigen binding fragment thereof by a gene recombination method, for example, appropriate host cells are transformed with the expression vector comprising the nucleic acid encoding the anti-COPB2 antibody or the antigen binding fragment thereof, and the obtained transformants are cultured under appropriate conditions to express the antibody or the antigen binding fragment. Then, isolation or purification may be performed by a method known in the art or a method equivalent thereto.

Examples of the method for isolating or purifying the antibody or the antigen binding fragment include, but are not particularly limited to, column purification using affinity columns with protein A or other chromatographic columns, filtration through filters, ultrafiltration, salting-out, and dialysis, which may be appropriately used in combination.

When the anti-COPB2 antibody has CDRs consisting of amino acid sequences derived from the amino acid sequences of heavy chain CDR1 to CDR3 and light chain CDR1 to CDR3 by the addition, substitution, or deletion of an amino acid or has CDRs consisting of amino acid sequences having 80% or higher identity to the amino acid sequences of heavy chain CDR1 to CDR3 and light chain CDR1 to CDR3, the anti-COPB2 antibody can be prepared by use of a method known in the art such as site-directed mutagenesis, random mutagenesis, chain shuffling, or CDR walking.

It is well known to those skilled in the art that antibodies or antigen binding fragments thereof having various mutations in CDRs are displayed on phage surface by a phage display method and screened using the antigen to obtain an antibody or an antigen binding fragment thereof comprising a CDR set with more matured affinity (e.g., Wu et al., PNAS, 95: 6037-6042 (1998); Schier, R. et al., J. Mol. Bio. 263: 551-567 (1996); Schier, R. et al., J. Mol. Biol. 255: 28-43 (1996); and Yang, W.P. et al., J. Mol. Biol., 254: 392-403 (1995)). Thus, the anti-COPB2 antibody may be prepared by this method.

A chimeric antibody, a humanized antibody, and a human antibody can be prepared by methods known in the art or methods equivalent thereto.

The chimeric antibody, the humanized antibody, and human antibody may be prepared by cloning an antibody gene from mRNA of a hybridoma producing a nonhuman animal antibody, linking this antibody gene to a portion of a human antibody gene by a gene recombination technique, and using the resulting gene.

The gene of the anti-COPB2 antibody may be determined depending on the amino acid sequences of the CDRs or the amino acid sequences of the variable regions described in (a) to (j).

The preparation of the chimeric antibody is not particularly limited, and the chimeric antibody may be produced using, for example, a nucleic acid obtained by substituting any of the heavy chain variable regions (VH) and the light chain variable regions (VL) (f) to (j) with human antibody-derived sequences by gene recombination.

Alternatively, the chimeric antibody may be obtained by cloning heavy chain and light chain constant regions of human IgG1, linking these human antibody-derived heavy chain and light chain constant regions to, for example, any of the heavy chain variable regions (VH) and the light chain variable regions (VL) (f) to (j), respectively, by an overlapping hanging method based on PCR, and inserting DNA obtained after amplification into an appropriate vector, which is then used in transformation.

In the preparation of the humanized antibody, human antibody variable domains are cloned and engineered so as to have the nucleotide sequences of the CDRs of the anti-COPB2 antibody by site-directed mutagenesis using a megaprimer method. If specific binding to the antigen can no longer be attained by humanizing amino acid sequences constituting framework regions, some amino acids of the framework regions may be converted from human type to rat type.

CDRs consisting of amino acid sequences differing by having deletion, substitution, or addition of one or more amino acids from the amino acid sequences of the original CDRs or CDRs consisting of amino acid sequences having 80% or higher identity to the original sequences may be prepared by use of a method known in the art such as site-directed mutagenesis, random mutagenesis, chain shuffling, or CDR walking.

The antigen binding fragment of the anti-COPB2 antibody may be expressed by the method mentioned above using the nucleic acid encoding the fragment, or the full-length antibody may be obtained and then fragmented by treatment with an enzyme such as papain or pepsin.

The anti-COPB2 antibody or the antigen binding fragment thereof obtained by the production method described above can be confirmed to be an anti-COPB2 antibody, for example, by using, as an index, the ability to detect the COPB2 protein in the sample by an immunological measurement method using the anti-COPB2 antibody or the antigen binding fragment thereof.

Immunoassay for detecting the protein of interest by immunological measurement is well known in the art.

Reagents necessary for each immunoassay are also well known. The COPB2 protein may be immunologically detected using the anti-COPB2 antibody or the antigen binding fragment thereof as an antibody for use in immunoassay, and using a usual immunoassay kit.

The immunoassay is not particularly limited and is preferably, for example, an immunohistochemical method (IHC), an immunofluorescent method (IF), flow cytometry, enzyme-linked immunosorbent assay (ELISA), or immunoblot.

### (Pharmaceutical composition)

The pharmaceutical composition for treating or preventing a cancer according to the present embodiment comprises an antibody against COPB2 or an antigen binding fragment thereof. In this context, the antibody against COPB2 or the antigen binding fragment thereof may be the same as that mentioned above.

The term "treatment or prevention" as used herein means a therapeutic and/or prophylactic procedure. The term "therapeutic or prophylactic procedure" is approved in the art and may include the administration of the pharmaceutical composition to a subject.

The therapeutic procedure may mean administration after finding of an undesirable condition (e.g., a disease or other undesirable conditions in a subject) and may intend to reduce, suppress, restore, alleviate, or stabilize an existing undesirable condition or an adverse reaction thereof. The therapeutic procedure also includes the attenuation of arbitrary direct or indirect pathological influence caused by a disease, and the prevention of metastasis.

The therapeutic procedure may also mean a procedure for delaying the onset of a disease or slowing down the progression of a disease.

The prophylactic procedure may mean administration before clinical finding of an undesirable condition and may intend to suppress and/or prevent the recurrence of an undesirable condition.

The term "pharmaceutical composition" as used herein may mean a pharmaceutical formulation or a pharmaceutical preparation that comprises an active ingredient and an additive (pharmaceutically acceptable component, etc.) and is generally used in the art. In the present embodiment, the pharmaceutical composition comprises at least an antibody against COPB2 or an antigen binding fragment thereof as an active ingredient.

One aspect of the present embodiment is a pharmaceutical composition for treating a cancer. The treatment of a cancer is not particularly limited and may mean the single-agent administration of or combination therapy using the pharmaceutical composition of the present embodiment resulting in decrease in the number of cancer cells in a subject, the disappearance of cancer cells, the suppression of the proliferation of cancer cells, decrease in tumor size, the suppression of the invasion of cancer cells to a peripheral organ, the suppression of the metastasis of cancer cells, or the improvement of various symptoms caused by the cancer, and preferably means the disappearance of cancer cells or the suppression of the metastasis of cancer cells.

One aspect of the present embodiment is a pharmaceutical composition for preventing a cancer. The prevention of a cancer is not particularly limited and may mean the suppression of cancer recurrence. The prevention of a cancer preferably means the prevention of increase in the number of cancer cells caused by the re-proliferation of cancer cells decreased in number, the prevention of the re-proliferation of cancer cells whose proliferation has been suppressed, or the prevention of re-increase in decreased tumor size.

The term "cancer" as used herein means a physiological condition in a mammal typically characterized by an unregulated cell growth or proliferation. Terms such as "cancer", "carcinoma", "tumor", and "neoplasm" are not discriminated from each other and are interchangeable with each other.

Examples of the cancer include, but are not particularly limited to, primary tumor, advanced or progressive cancer, recurrent cancer, metastatic cancer, and combinations thereof. The pharmaceutical composition is more preferably used in the treatment or prevention of primary tumor.

The term "recurrence" as used herein means that a cancer develops again after a continuous period for which no cancer is detected, a cancer reduced in size by treatment glows large again, or the same cancer appears at another site.

The term "metastasis" as used herein means that cancer cells move from their original tumor site to another site of an organ via a vascular vessel or lymph and increase in number at the site.

The cancer may be a cancer developed in any organ or tissue and is preferably a cancer developed in an organ or a tissue having distributed COPB2. Examples of the cancer include, but are not particularly limited to, cancers that develop in the brain, the eyes, endocrine tissues, the respiratory system, the gastrointestinal tract, the liver, the gallbladder, the pancreas, the kidney, the urinary bladder, reproductive tissues, muscular tissues, connective tissues, soft tissues, the skin, the bone marrow, and lymph tissues. The cancer may be any of epithelial malignant tumor and nonepithelial malignant tumor.

Specific examples of the cancer include, but are not particularly limited to, thyroid gland cancer, adrenocortical cancer (ACC), lung cancer (e.g., small-cell lung cancer, non-small cell lung cancer, lung adenocarcinoma (LUAD), and squamous cell carcinoma of the lung), gastrointestinal cancer (e.g., esophageal cancer, stomach cancer, large intestinal cancer, and rectal adenocarcinoma (READ)), cholangiocarcinoma (CHOL), liver cancer, hepatocellular carcinoma, hepatoma, pancreatic cancer, kidney cancer (e.g., kidney chromophobe carcinoma (KICH) and kidney renal papillary cell carcinoma (KIRP)), urinary bladder cancer, peritoneal cancer, salivary gland cancer, skin cancer (e.g., skin cutaneous melanoma (SKCM)), squamous cell carcinoma, multiple myeloma, glioma, brain tumor (e.g., low-grade glioma (LGG) and glioblastoma multiforme (GBM)), pheochromocytoma and paraganglioma (PCPG), uterine cancer (e.g., endometrial cancer, uterine corpus cancer, uterine cervical cancer, and uterine carcinosarcoma (UCS)), ovary cancer (e.g., ovarian serous cystadenocarcinoma (OV)), breast cancer (e.g., breast adenocarcinoma and breast invasive carcinoma (BRCA)), prostate cancer, testis cancer (e.g., testicular germ cell tumor (TGCT)), vulval cancer, thymoma (THYM), leukemia (e.g., AML (acute myeloid leukemia) and CML (chronic myeloid leukemia)), malignant lymphoma, ALL (acute lymphatic leukemia), CLL (chronic lymphatic leukemia), Hodgkin lymphoma, non-Hodgkin lymphoma, lymphoid neoplasm diffuse large B-cell lymphoma (DLBC), and other lymphoproliferative disorders.

Among them, the pharmaceutical composition of the present embodiment is preferably used in the treatment or prevention of one or more cancers selected from the group consisting of breast cancer, glioma, gastrointestinal cancer, hepatocellular carcinoma, liver cancer, prostate cancer, lung cancer, and pancreatic cancer, and more preferably used in the treatment or prevention of one or more cancers selected from the group consisting of breast cancer, liver cancer, stomach cancer, large intestinal cancer, hepatocellular carcinoma, prostate cancer, lung cancer, and pancreatic cancer.

The pharmaceutical composition of the present embodiment comprises the anti-COPB2 antibody or the antigen binding fragment thereof and may further comprise a pharmaceutically acceptable carrier and/or an additive. The blending ratio of the carrier or the additive can be appropriately set on the basis of a range usually adopted in the field of medicaments.

Examples of the carrier include, but are not particularly limited to, water, saline, other aqueous solvents, and aqueous or oil bases.

Examples of the additive include, but are not particularly limited to, excipients, binders, pH adjusters, disintegrants, absorption promoters, lubricants, colorants, corrigents, and flavors.

Examples of the form of formulation of the pharmaceutical composition include, but are not particularly limited to: oral agents such as tablets, coated tablets, pills, powders, granules, capsules, solutions, suspension, and emulsions; and parenteral agents such as injections, transfusions, suppositories, ointments, and patches.

A method for administering the pharmaceutical composition is not particularly limited, the pharmaceutical composition is preferably administered as an injection or a transfusion through a parenteral administration route, for example, intravenously, intramuscularly, intradermally, intraperitoneally, subcutaneously, or locally, and more preferably administered locally.

The injection or the transfusion comprising the anti-COPB2 antibody or the antigen binding fragment thereof can be used as a solution, a suspension or an emulsion. Examples of the solvent therefor include injectable distilled water, saline, glucose solutions, and isotonic solutions (e.g., sodium chloride, potassium chloride, glycerin, mannitol, sorbitol, boric acid, borax, and propylene glycol solutions). One of these solvents may be used singly, or two or more thereof may be used in combination.

The injection or the transfusion may further contain an additive such as a stabilizer, a solubilizer, a suspending agent, an emulsifying agent, a soothing agent, a buffer, a preservative, an antiseptic, or a pH adjuster.

Examples of the stabilizer include, but are not particularly limited to, albumin, globulin, gelatin, mannitol, glucose, dextran, ethylene glycol, propylene glycol, ascorbic acid, sodium bisulfite, sodium thiosulfate, sodium EDTA, sodium citrate, and dibutylhydroxytoluene.

Examples of the solubilizer include, but are not particularly limited to, alcohols (e.g., ethanol), polyalcohols (e.g., propylene glycol and polyethylene glycol), and nonionic surfactants (e.g., Polysorbate 80(R) and HCO-50).

Examples of the suspending agent include, but are not particularly limited to, glycerin monostearate, aluminum monostearate, methylcellulose, carboxymethylcellulose, hydroxymethylcellulose, and sodium lauryl sulfate.

Examples of the emulsifying agent include, but are not particularly limited to, gum arabic, sodium alginate, and tragacanth.

Examples of the soothing agent include, but are not particularly limited to, benzyl alcohol, chlorobutanol, and sorbitol.

Examples of the buffer include, but are not particularly limited to, phosphate buffer solutions, acetate buffer solutions, borate buffer solutions, carbonate buffer solutions, citrate buffer solutions, and Tris buffer solutions.

Examples of the preservative include, but are not particularly limited to, methyl p-hydroxybenzoate, ethyl p-hydroxybenzoate, propyl p-hydroxybenzoate, butyl p-hydroxybenzoate, chlorobutanol, benzyl alcohol, benzalkonium chloride, sodium dehydroacetate, sodium edetate, boric acid, and borax.

Examples of the antiseptic include, but are not particularly limited to, benzalkonium chloride, p-hydroxybenzoic acid, and chlorobutanol.

Examples of the pH adjuster include, but are not particularly limited to, hydrochloric acid, sodium hydroxide, phosphoric acid, and acetic acid.

The dose and dosing interval of the active ingredient in the pharmaceutical composition can be appropriately selected depending on a recipient, an administration route, a disease, and the age, body weight, and symptoms of the subject.

For example, in the case of oral administration, the dose of the active ingredient to an adult is, for example, but not particularly limited to, 0.01 mg to 10 g per day and can be 100 mg to 6 g or can be 50 mg to 500 mg. As for the dosing interval of the pharmaceutical composition, the daily dose may be administered once a day or may be administered in several divided portions.

The subject to which the pharmaceutical composition is administered is a mammal. Examples of the mammal include, but are not particularly limited to, humans, nonhuman primates, domestic animals, laboratory animals, and livestock. A human is preferred.

The pharmaceutical composition of the present embodiment may be administered in combination with one or more additional agents. Examples of the additional agent include, but are not particularly limited to: other therapeutic agents such as immunomodulators, cell division inhibitors, cell adhesion inhibitors, cytotoxic agents, cell apoptosis activators, and agents increasing the sensitivity of cells to apoptosis inducing factors; and anticancer agents such as microtubule disrupting agents, antimetabolic agents, topoisomerase inhibitors, DNA intercalators, alkylating agents, hormone therapy, kinase inhibitors, receptor antagonists, tumor cell apoptosis activators, and anti-angiogenic agents.

For combined administration, the pharmaceutical composition of the present embodiment may be used separately from the additional agent.

For concurrent administration, two or more active ingredients may be contained in the same preparation or may be separately administered as different preparations.

For separation administration, two or more active ingredients can be administered in accordance with desired administration regimens. The dose and dosing interval of the additional active ingredient can abide by a predetermined administration regimen.

### Examples

Some examples of the embodiments of the present application will be described below. Hereinafter, the present invention will be specifically described with reference to Examples. However, the present invention is not limited by these Examples

### 1. Preparation of antigen protein

cDNA was synthesized such that a 6 x His tag is connected downstream of cDNA encoding 255 amino acid residues corresponding to glutamine at position 650 to aspartic acid at position 906 in the amino acid sequence of human COPB2. This cDNA was cloned into an expression vector pcDNA5/TO (Thermo Fisher Scientific Inc.). Expi293 cells (Thermo Fisher Scientific Inc.) were transfected with this plasmid so that human COPB2 antigen protein was expressed. The transfection was performed using Expi293fectamine (Thermo Fisher Scientific Inc.) in accordance with the method described in the attached document. A culture solution and the cells were each recovered 6 days after transfection, and a cell extract was prepared from the cells using RIPA (Invitrogen Corp.). 10 µL of the culture solution and 10 µg of the cell extract were subjected to SDS-PAGE under reducing conditions, and the expression of COPB2(650-906) was confirmed by Western blot using an anti-His tag antibody (Proteintech Group, Inc.).

As a result, the expression of COPB2(650-906) was found in both the culture solution and the cells, and it was confirmed that more COPB2(650-906) was expressed and accumulated in the cells.

The purification of the COPB2(650-906) protein from the culture solution of the transfected Expi293 cells and the cells was carried out using TALON resin (Clontech Laboratories, Inc.). By two transfection experiments, 524 µg in total of purified COPB2(650-906) protein was obtained from the culture solution and the cells.

### 2. Preparation of monoclonal antibody

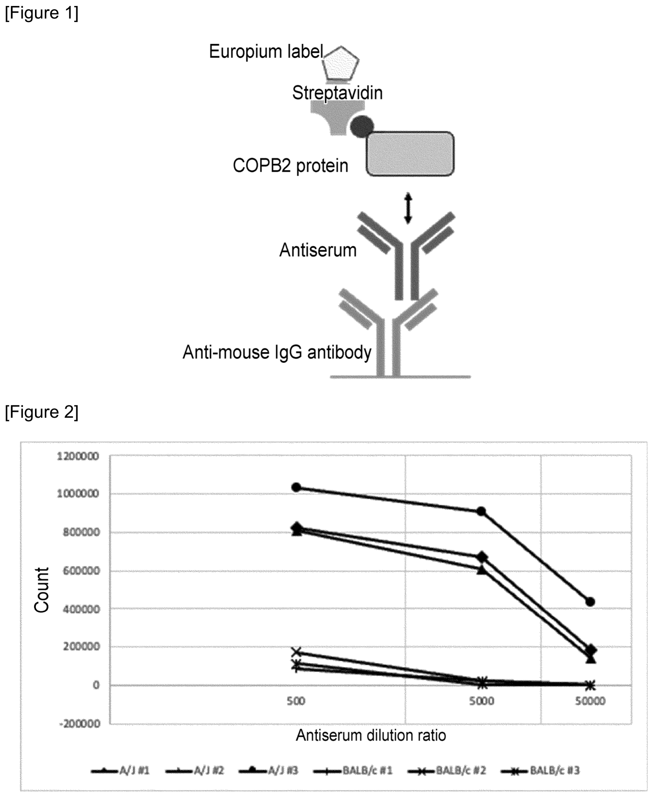

Three A/Jackson mice and three Balb/c mice were immunized with the purified human COPB2(650-906) protein. Specifically, 50 µg/shot was administered intraperitoneally and 50 µg/shot was administered subcutaneously to the neck per mouse. This immunization was carried out twice every three weeks. After the two immunizations, blood was collected, and an antibody titer against the antigen protein was measured. The principle of the measurement method is shown in Figure 1.

As a result, a high antibody titer of 800000 counts or more was found in all the A/Jackson mice immunized with the human COPB2 protein. On the other hand, an antibody titer of 200000 counts or less was found in the Balb/c mice (Figure 2).

One A/Jackson mouse (A/J#3 mouse) that exhibited a high antibody titer after two immunizations with the purified human COPB2 protein was given the antigen protein as a final booster, and the spleen was excised 4 days later. Spleen cells were prepared and subsequently subjected to cell fusion with myeloma cells (P3U1). Hybridomas were inoculated to ten 96-well plates, and an antibody titer against the immunogen human COPB2 protein was measured for culture supernatants in a total of 928 wells. As a result, five positive samples were confirmed. The cells contained in the wells of these five positive samples were further subjected to the limiting dilution method twice to obtain monoclonal lines. As a result, hybridomas of two clones were established as cell lines producing antibodies that exhibited high reactivity with the COPB2 protein (clone #A3-3G10 and #A3-8C9).

Further, spleen cells were also prepared from the mouse (A/J#2 mouse) that proceeded to the third immunization and exhibited a high antibody titer, and subjected to cell fusion in the same manner as above. The screening of hybridomas of 768 specimens was carried out. As a result, two positive samples were detected, each of which was further subjected to the limiting dilution method twice to obtain a monoclonal line (clone #A2-7C6 and #A2-7D11).

In this way, anti-COPB2 monoclonal antibodies of a total of four clones were prepared.

### 3. Study on Western blot

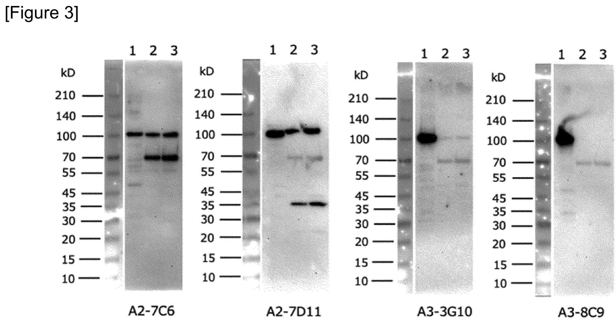

A study was made on whether the antibodies of four clones could detect the COPB2 protein in human cells and mouse cells by Western blot. Specifically, 10 µg of a cell extract prepared from human-derived HEK293 cells with RIPA lysis solution (manufactured by Santa Cruz Biotechnology, Inc.) and 5 or 10 µg of a tissue extract prepared from the mouse liver with T-PER lysis solution (manufactured by Thermo Fisher Scientific Inc.) were each subjected to SDS-PAGE under reducing conditions and then transferred to Immobilon-P membrane (Merck Millipore), which was then blocked in TBS-T (0.05 M Tris-HCl, 0.15 M NaCl, and 0.05% Tween 20, pH 7.6) containing 3% skim milk at room temperature for 1 hour and reacted overnight at 4°C with a solution of each antibody dissolved at a concentration of 1 µg/mL in TBS-T containing 3% skim milk. Each membrane thus reacted was washed three times with TBS-T for 10 minutes, then reacted at 4°C for 2 hours with a solution of HRP-labeled rabbit anti-mouse IgG polyclonal antibody (manufactured by Dako) diluted 1000-fold with TBS-T containing 3% skim milk, and washed three times with TBS-T for 10 minutes, followed by detection using Chemi-Lumi One Super (manufactured by Nacalai Tesque, Inc.) (Figure 3). As a result, all the antibodies reacted strongly with human COPB2, and it was suggested that clone #A2-7C6 and #A2-7D11 also cross-reacted with mouse COPB2. Also, clone #A3-3G10 weakly cross-reacted with mouse COPB2, and clone #A3-8C9 had no reaction with mouse COPB2.

### 4. Structural analysis of anti-COPB2 antibody

The heavy chain and light chain variable regions of the anti-COPB2 monoclonal antibodies A2-7C6, A2-7D11, and A3-8C9 were structurally analyzed with Bioinformatic Analysis System of Repertoire Genesis Inc.

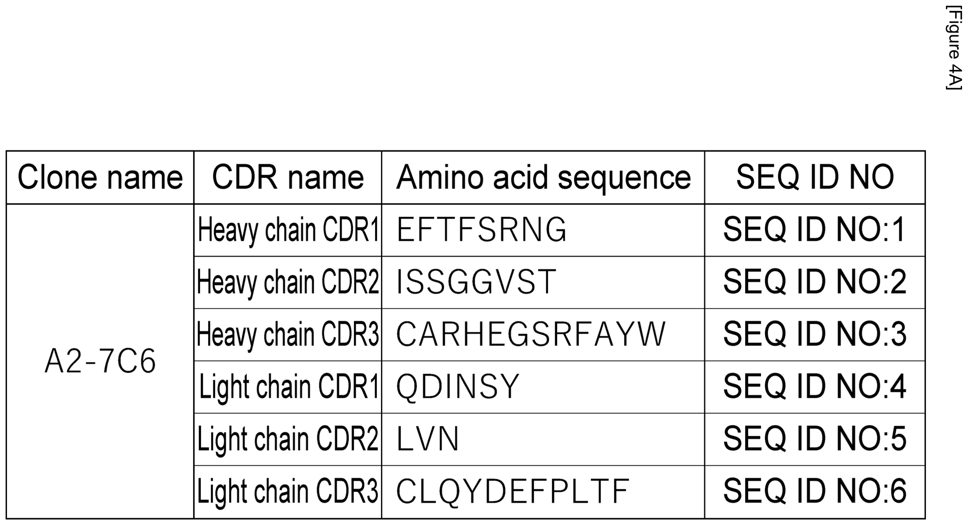

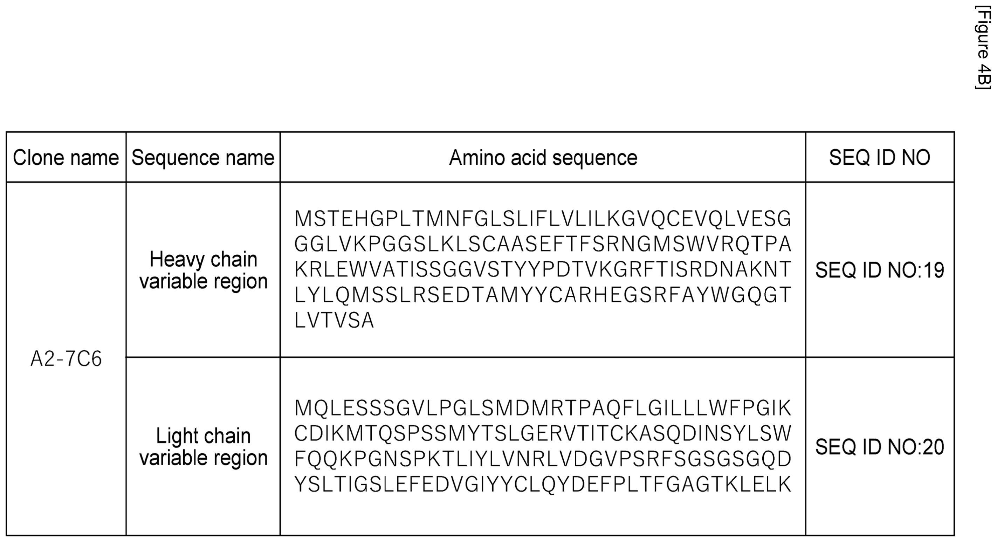

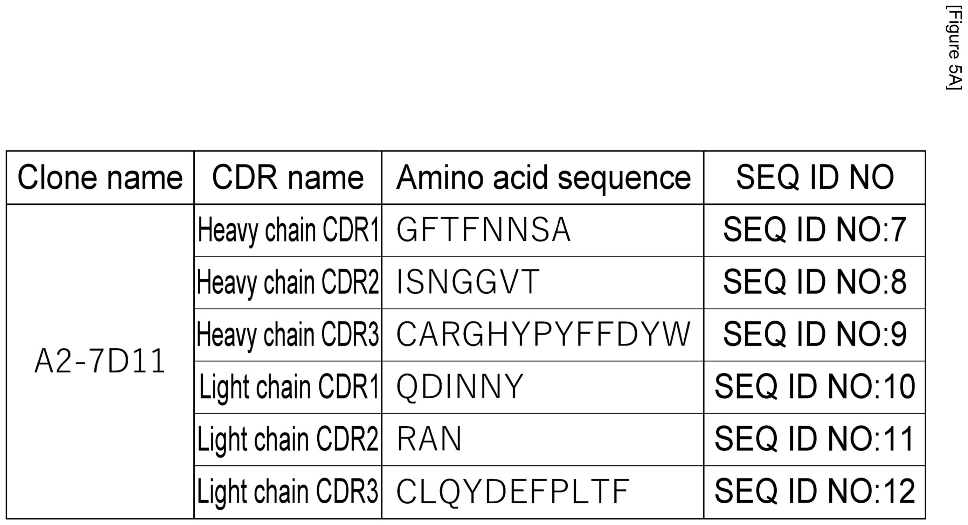

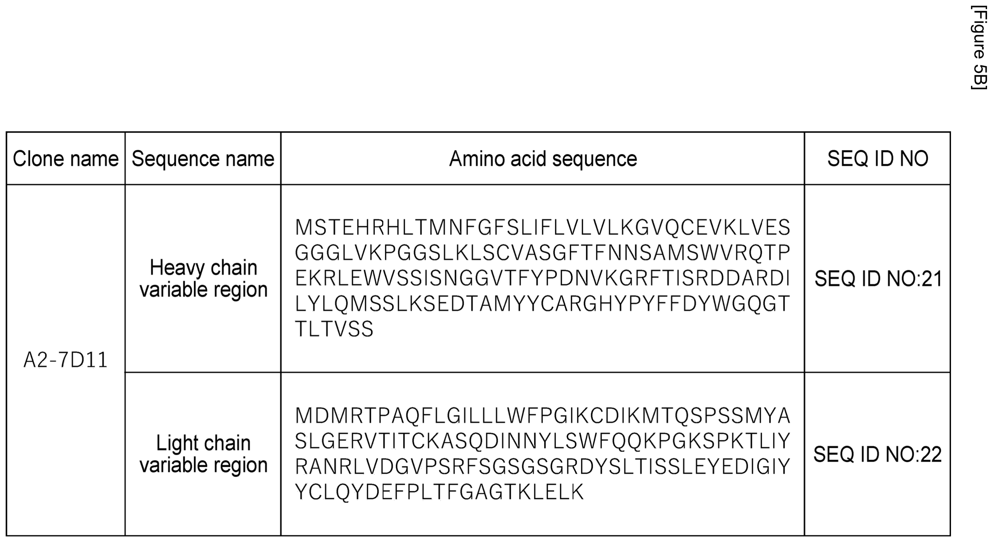

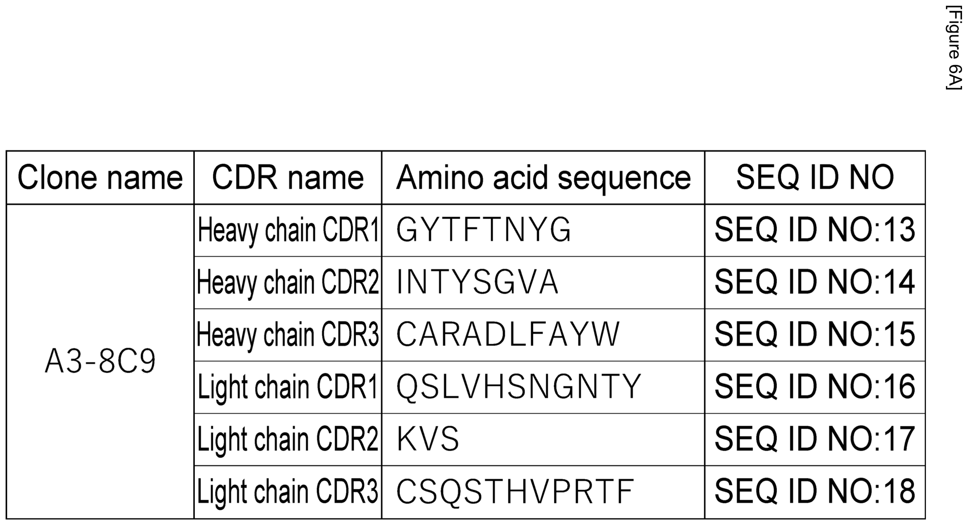

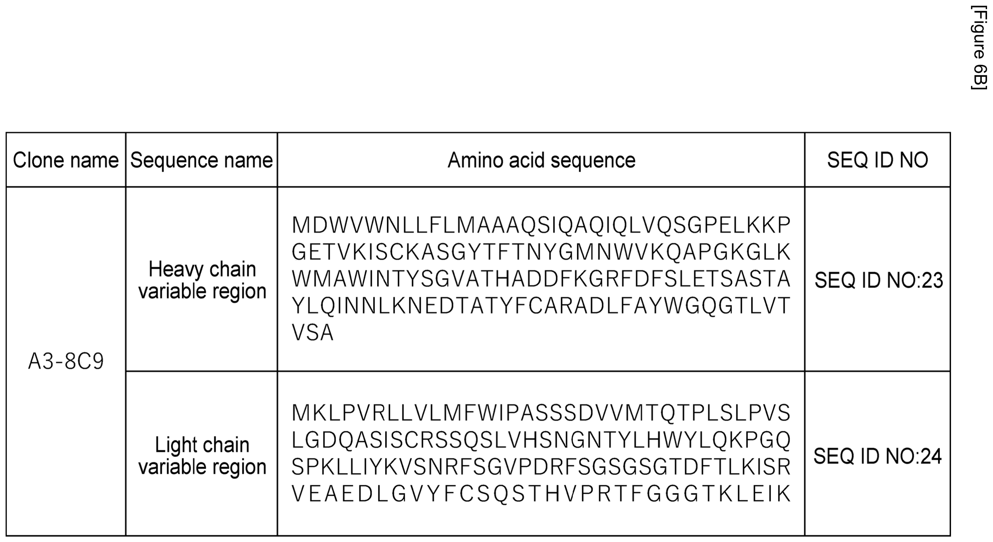

Figures 4 to 6 show the amino acid sequences of heavy chain CDR1 to CDR3 and light chain CDR1 to CDR3 and the amino acid sequences of the heavy chain and light chain variable regions in the anti-COPB2 monoclonal antibodies A2-7C6, A2-7D11, and A3-8C9.

### 5. Preparation of human monoclonal antibody

### a: Induction of antibody by immunization

Five TC-mAb mice (Trans Chromosomics, Inc.) (individual Nos: 7337, 7366, 7397, 7412, and 7484) were immunized with the purified human COPB2 (650-906) protein. Immunization locations were a plurality of sites per mouse, and administration was carried out two or more times within 3 weeks. After a lapse of 3 weeks, blood was collected, and an antibody titer in serum was confirmed by ELISA.

The experimental procedures of ELISA are as follows: the antigen protein was dispensed at 50 ng/well to Immunoplate Maxisorp (442404, Thermo Fisher Scientific Inc.) and immobilized thereon. Then, the plate was blocked with 5% skim milk (Difco, 232100)/TBS-T (50 mM Tris-HCl (pH 7.4), 0.14 M NaCl, 0.05% Tween 20). Then, the serum diluted at 1/2,000, 1/8,000, 1/32,000, and 1/128,000 with TBS-T was added thereto and reacted. Subsequently, Anti-IgG(Fc), Human, Goat-Poly, HRP (A80-104P, Bethyl Laboratories, Inc.) diluted at 1/50,000 with TBS-T was added thereto and reacted. Then, color was developed with 0.5 mg/mL o-phenylenediamine, and the reaction was terminated with 1 M sulfuric acid. Finally, absorbance at 492 nm was measured, and an antibody titer was calculated.

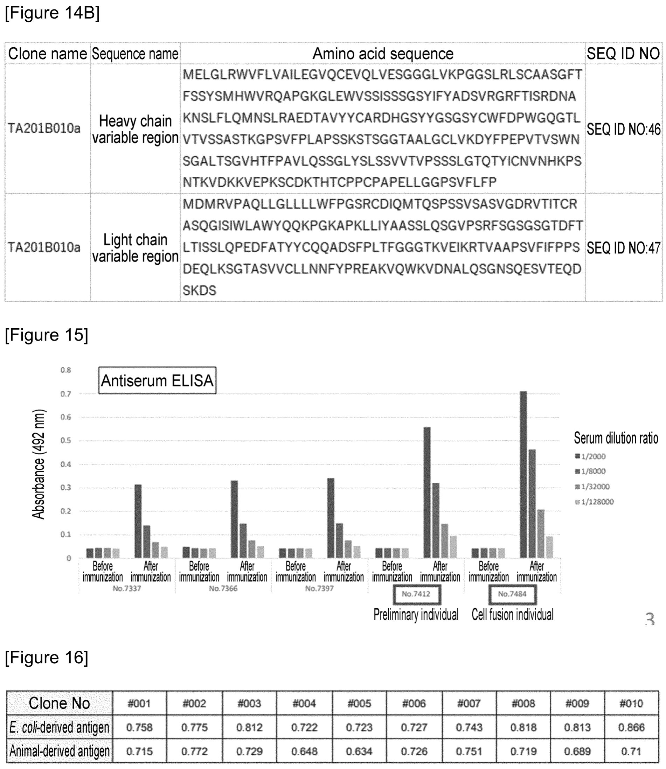

As a result, elevation in antibody titer was confirmed in all the immunized individuals (Figure 15). Of the five animals, an individual having the highest antibody titer (individual No: 7484) was used in the experiment of cell fusion.

### b: Cell fusion

Internal cells were recovered from the spleen, iliac lymph nodes, and the like of individual No: 7484, and the number of cells was counted. The cells were suspended in an electrode solution for electric fusion (0.3 M mannitol, 0.1 mM CaCl₂, 0.1 mM MgCl₂) and mixed with the same number of myeloma cells (P3X63Ag8.653) suspended in the same electrode solution for electric fusion as above. The cells were fused using a cell fusion apparatus (ECFG21, Nepa Gene Co., Ltd.), inoculated to a 96-well plate with a medium containing HAT (H0262-10VL, Merck Millipore), and selectively cultured for 2 weeks.

### c: Screening

The antigen protein expressed in E. *coli* was dispensed at 50 ng/well to Immunoplate Maxisorp (442404, Thermo Fisher Scientific Inc.) and immobilized thereon. The plate was blocked with 5% skim milk (Difco, 232100)/TBS-T (50 mM Tris-HCl (pH 7.4), 0.14 M NaCl, 0.05% Tween 20). Then, the hybridoma culture supernatant diluted at 2/3 with TBS-T was added thereto and reacted. Subsequently, Anti-IgG(Fc), Human, Goat-Poly, HRP (A80-104P, Bethyl Laboratories, Inc.) diluted at 1/50,000 with TBS-T was added thereto and reacted. Color was developed with 0.5 mg/mL o-phenylenediamine, and the reaction was terminated with 1 M sulfuric acid. Finally, absorbance at 492 nm was measured to perform screening (primary screening).

Hybridoma colonies were picked up from positive wells of the primary screening. After culture for 2 to 3 days, culture supernatants were recovered and screened by ELISA in the same manner as in the primary screening (secondary screening).

### d: Cloning

Hybridoma cells of top 10 clones of the absorbance obtained by the secondary screening were cloned by use of the limiting dilution method. Positive wells were screened by ELISA in the same manner as in the primary screening. Cloning was repeated until a positive well of a single colony was able to be obtained two or more times. Of 147 positive hybridomas, 6 clones having a high antibody titer (identification Nos: #002, #003, #007, #008, #009, and #010) were used in antibody purification (Figure 16).

### e: Antibody purification

The hybridoma cells of identification Nos: #002, #003, #007, #008, #009, and #010 were each cultured in 100 mL of a serum-free medium for 2 weeks, and culture supernatants were recovered. The antibody purification was carried out using Protein G Sepharose (Cytiva) in accordance with the method described in the attached document. The antibodies thus purified were concentrated using an ultrafiltration membrane while the solvent was replaced with DPBS(-).

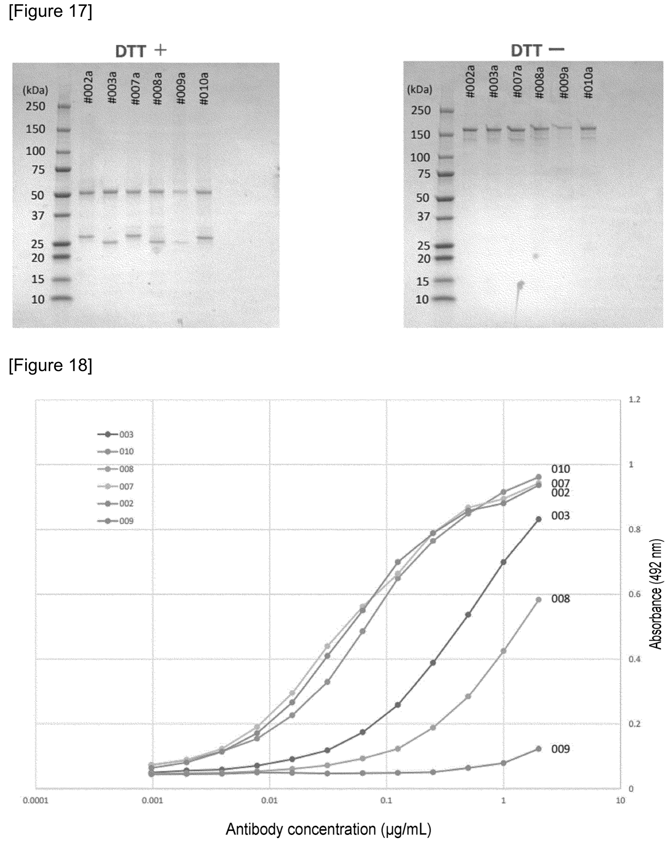

The antibody purification was confirmed by SDS-PAGE (Figure 17). Antibody titers were further confirmed by ELISA (Figure 18). Clones of identification Nos: #002, #003, #007, #008, and #010 included in top 5 clones of the antibody titer were used in Western blot analysis.

### 6. Study on Western blot

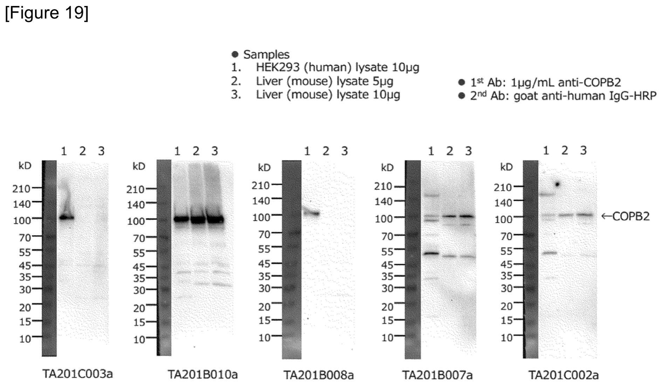

A study was made on whether the antibodies of five clones could detect the COPB2 protein in human cells and mouse cells by Western blot. Specifically, 10 µg of a cell extract prepared from human-derived HEK293 cells with RIPA lysis solution (manufactured by Santa Cruz Biotechnology, Inc.) and 5 or 10 µg of a tissue extract prepared from the mouse liver with T-PER lysis solution (manufactured by Thermo Fisher Scientific Inc.) were each subjected to SDS-PAGE under reducing conditions and then transferred to Immobilon-P membrane (Merck Millipore), which was then blocked in TBS-T (0.05 M Tris-HCl, 0.15 M NaCl, and 0.05% Tween 20, pH 7.6) containing 3% skim milk at room temperature for 1 hour and reacted overnight at 4°C with a solution of each antibody dissolved at a concentration of 1 µg/mL in TBS-T containing 3% skim milk. Each membrane thus reacted was washed three times with TBS-T for 10 minutes, then reacted at 4°C for 2 hours with a solution of HRP-labeled rabbit anti-mouse IgG polyclonal antibody (manufactured by Dako) diluted 1000-fold with TBS-T containing 3% skim milk, and washed three times with TBS-T for 10 minutes, followed by detection using Chemi-Lumi One Super (manufactured by Nacalai Tesque, Inc.) (Figure 19). As a result, all the antibodies reacted strongly with human COPB2, and it was suggested that clones other than identification Nos: #003 and #008 also cross-reacted with mouse COPB2. Also, the clone of identification No: #010 was confirmed to react strongly with both human COPB2 and mouse COPB2. The clones of identification Nos: #002 and #007 were confirmed to react more strongly with mouse COPB2 than with human COPB2.

### 7. Isotype analysis and CDR analysis

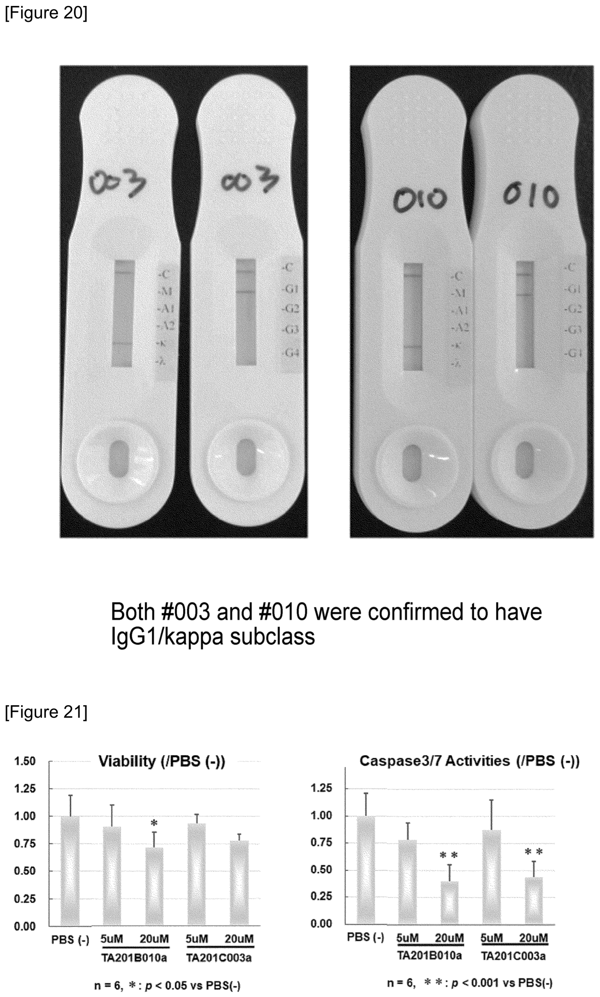

The procedures of isotype analysis are as follows: the purified antibodies of identification Nos: #003 and #010 were each diluted into 2 ug/mL with D-PBS(-) and added at 100 uL/cassette to a human isotyping kit (ISO-Hu-20, Antagen Biosciences, Inc.). As a result, the clones of identification Nos: #003 and #010 were confirmed to have IgG1/kappa subclass (Figure 20).

The procedures of CDR analysis are as follows: the hybridoma cells of identification Nos: #003 and #010 were each cultured in a 10 cm dish until confluent. Then, cell pellets were recovered by centrifugation and subjected to RNA extraction. The RNA extraction was carried out using NucleoSpinRNA Plus (Takara Bio Inc.) in accordance with the method described in the attached document. The extracted RNA was reverse-transcribed using SMARTer RACE 5'/3' Kit (Takara Bio Inc.) in accordance with the method described in the attached document to obtain cDNA. The obtained cDNA was ligated with a pRACE sequence (Takara Bio Inc.) to prepare an expression vector. The obtained expression vector was cloned in E. *coli* in accordance with a routine method, and CDR sequences were analyzed by the sequence analysis of a purified plasmid.

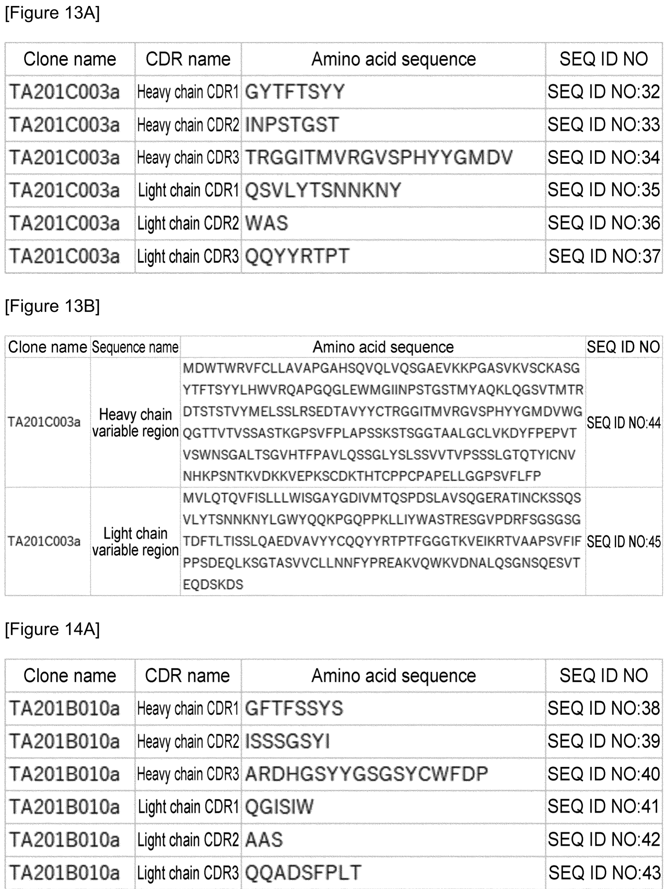

Figures 13 and 14 each show the amino acid sequences of heavy chain CDR1 to CDR3 and light chain CDR1 to CDR3 and the amino acid sequences of a heavy chain variable region and a light chain variable region of the human monoclonal antibody #003 or #010.

### 8. Study on administration of anti-COPB2 antibody to breast cancer cell

### a) Study on cytocidal effect (cell viability) and apoptosis inducing effect (caspase 3/7 activity)

A human triple negative breast cancer cell line MDA-MB-231-luc-D3H2LN (Xenogen, Inc.) was inoculated at a density of 2,000 cells/well to a 96-well plate and cultured in 0.5 mL of a serum-free medium. After 24 hours from the start of culture, the medium was replaced with a medium containing a complete human monoclonal antibody TA201B010a or TA201C003a at a concentration of 5 µM or 20 µM or a medium containing PBS(-) as a control. After 72 hours, cell viability and caspase 3/7 activity of the cultured cells were measured using ApoLive-Glo Multiplex Assay kit in accordance with the method described in the attached document.

### b) Results

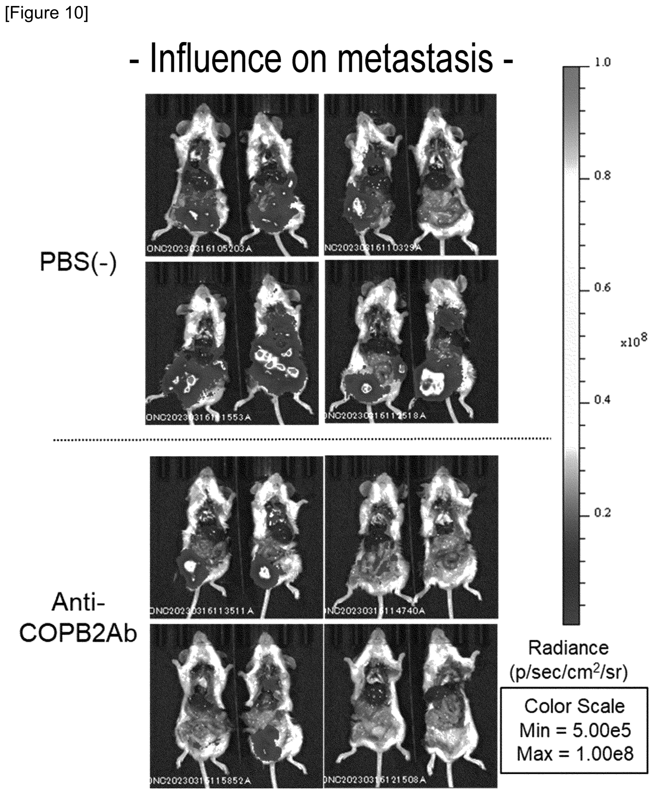

The presence of the complete human monoclonal antibody (TA201B010a clone) at a concentration of 20 µM was confirmed to cause significant reduction in viability in the human breast cancer cells (MDA-MB-231-luc-D3H2LN) (Figure 10). On the other hand, significant reduction in caspase 3/7 was also confirmed in the presence of the TA201B010a clone and the TA201C003a clone both at a concentration of 20 µM (Figure 10). This suggests that the anti-COPB2 antibodies had an inducing effect on cell death for breast cancer cells, though the cell death was presumed to be ascribable to a process independent from caspase activity.

### 9. Study on administration of anti-COPB2 antibody to breast cancer model mouse

### a) Preparation of breast cancer model mouse

A human triple negative breast cancer cell line MDA-MB-231-luc-D3H2LN (Xenogen, Inc) was adjusted to 8 × 10⁷ cells + 2 ml (0.5 ml of PBS:1.5 ml of MatriMix) and 4 × 10⁷ cells/ml (MatriMix was prepared on ice so as to attain A solution:B solution:C solution = 5.4:0.6:4.0) and transplanted at 2 × 10⁶ cells to subcutaneous adipose tissues around the inguinal papilla of Scid-Beige mouse (Jackson Laboratory Japan, Inc., female, 6 weeks old) to prepare a breast cancer model mouse.

### b) Administration of anti-COPB2 antibody

The anti-COPB2 antibody (anti-COPB2 monoclonal antibody A2-7C6) was administered at 50 µg/0.1 mL/mouse to the tail vein 5, 8, 11, 14, and 17 days after cell transplantation. The same volume of PBS was administered as a control (n = 8).

### c) Evaluation 1

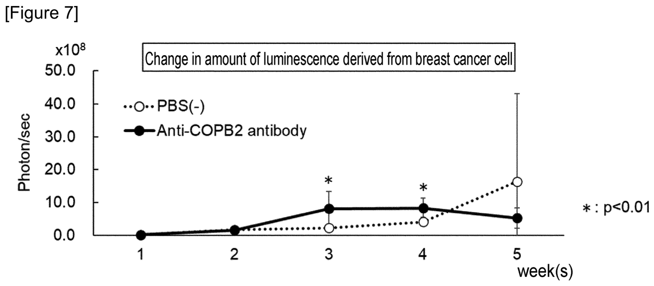

In order to examine change in amount of luminescence derived from breast cancer cells, measurement was performed using IVIS (PerkinElmer, Inc., IVIS Lumina-LT) 7, 14, 21, 28, and 37 days after transplantation. Images were taken under conditions involving FOV (field of view) of 12.5, f1.2, a measurement time of 30 seconds, and binning of 8 or 4. Living Image, ver. 4.7.3 was used in analysis to quantify the amount of luminescence. The results are shown in Figure 7. The amount of systemic luminescence in the mice measured with IVIS every week from the transplantation of human breast cancer cells was significantly higher in the anti-COPB2 antibody administration group than in the PBS(-) administration group on weeks 3 and 4, whereas the amount of luminescence was reversed and tended to be low in the anti-COPB2 antibody administration group on week 5.

### d) Evaluation 2

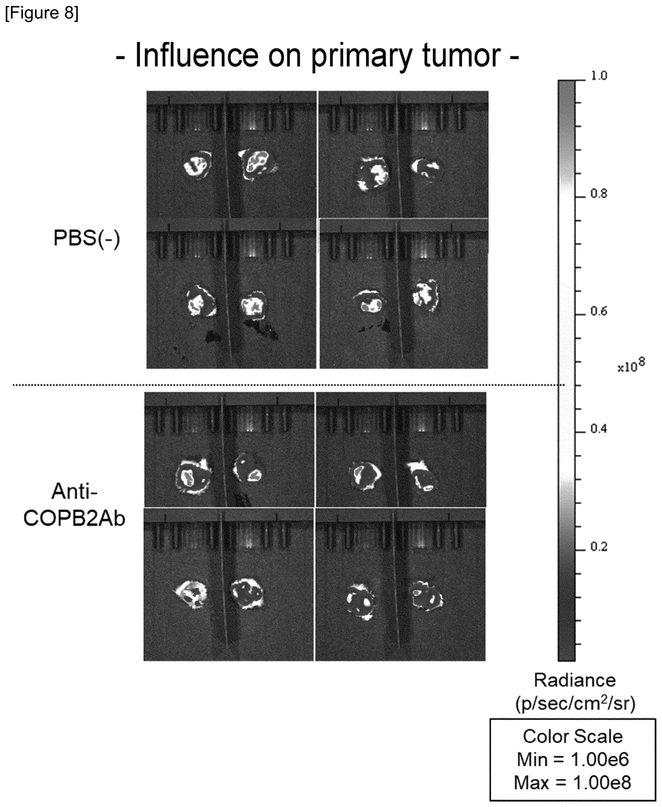

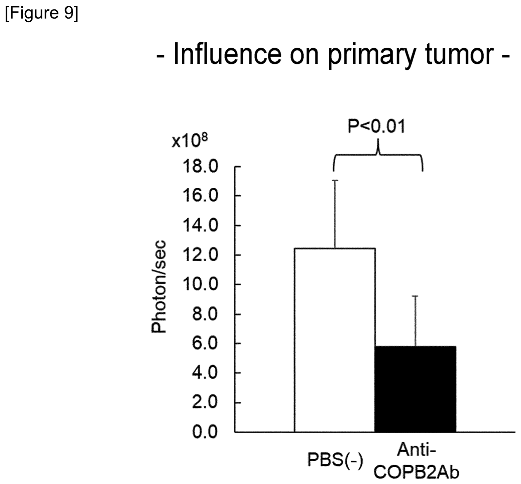

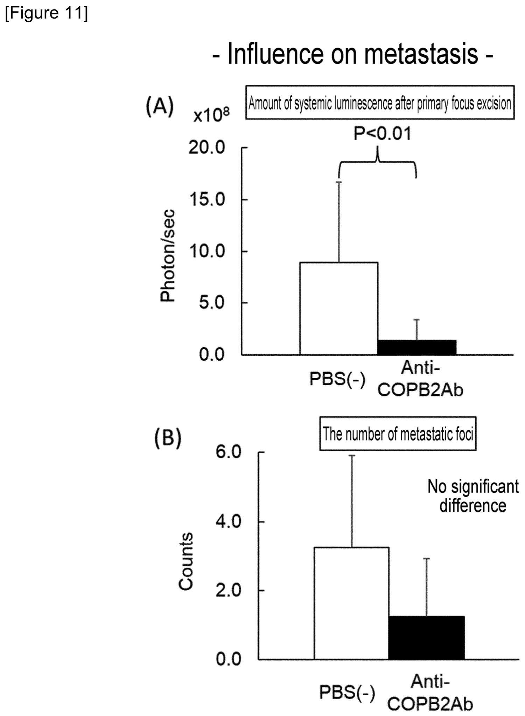

The breast cancer model mice were euthanized on week 5 (day 37), and primary tumor was excised. The amount of luminescence of the primary tumor and the amount of systemic luminescence of the breast cancer model mice after primary tumor excision were measured. The results are shown in Figures 8 to 11. The amount of luminescence of the primary tumor and the amount of systemic luminescence of the breast cancer model mice after primary tumor excision were significantly low in the results obtained about the anti-COPB2 antibody administration group, and the number of metastatic foci also tended to be decreased. These results demonstrated that the administration of the anti-COPB2 antibody reduced the size of the transplanted primary tumor and suppressed cancer metastasis.

## Claims

1. A pharmaceutical composition for treating or preventing a cancer, comprising an antibody against coatomer protein complex subunit beta 2 (COPB2) or an antigen binding fragment thereof.

2. The pharmaceutical composition according to claim 1, wherein the cancer is primary tumor.

3. The pharmaceutical composition according to claim 1 for suppressing cancer recurrence.

4. The pharmaceutical composition according to claim 1 for suppressing cancer metastasis.

5. The pharmaceutical composition according to claim 1, wherein the cancer is one or more members selected from the group consisting of breast cancer, glioma, gastrointestinal cancer, hepatocellular carcinoma, liver cancer, prostate cancer, lung cancer, and pancreatic cancer.
